# EUROPEAN PATENT APPLICATION

(11) **EP 3 845 638 A1**
(43) Date of publication of application: **07.07.2021**
(21) Application number: 19842326.1
(22) Date of filing: 26.07.2019
(51) Int. Cl.: C12N 5/10, A61K 35/17, C12N 5/0783, C12N 5/0789, A61K 35/545, A61P 31/00, A61P 37/06, A61P 37/08, C12N 15/09, C12N 15/12, C12N 15/85

(54) **METHOD FOR PRODUCING FOREIGN ANTIGEN RECEPTOR GENE-INTRODUCED CELL**

(30) Priority: 26.07.2018 JP 2018140523
(71) Applicant: Kyoto University, Kyoto-shi, Kyoto 606-8501 (JP); National University Corporation Shiga University of Medical Science, Otsu-shi Shiga 520-2192 (JP)
(72) Inventor: KAWAMOTO, Hiroshi, Kyoto-shi, Kyoto 606-8501 (JP); AGATA, Yasutoshi, Otsu-shi, Shiga 520-2192 (JP); NAGANO, Seiji, Kyoto-shi, Kyoto 606-8501 (JP); TERADA, Koji, Otsu-shi, Shiga 520-2192 (JP); MASUDA, Kyoko, Kyoto-shi, Kyoto 606-8501 (JP)
(74) Representative: Boult Wade Tennant LLP
(86) International application number: PCT/JP2019/029537
(87) International publication number: WO 2020/022512

(57) **Abstract**

Provided is a method for producing a cell incorporating an antigen specific receptor gene, which comprises the step of introducing an exogenous TCR or CAR gene into a material cell so that the introduced gene is expressed under the T cell receptor expression control system of the material cell.

SEQUENCE LISTING

<110>  Kyoto University
       National University Corporation Shiga University of Medical
        Science

<120>  Method for Generating Cells Incorporated With a Gene encording
       for Antigen Receptor

<130>  675044

<150>  JP2018-140523
<151>  2018-07-26

<160>  51

<170>  PatentIn version 3.5

<210>  1
<211>  34
<212>  DNA
<213>  Artificial Sequence

<220>
<223>  Cre recombinase target sequence lox2272

<400>  1
ataacttcgt ataaagtatc ctatacgaag ttat                                  34


<210>  2
<211>  34
<212>  DNA
<213>  Artificial Sequence

<220>
<223>  Cre recombinase target sequence loxP

<400>  2
ataacttcgt atagcataca ttatacgaag ttat                                  34


<210>  3
<211>  70
<212>  DNA
<213>  Artificial Sequence

<220>
<223>  Primer

<400>  3
gcggtggcgg ccgctataac ttcgtataaa gtatcctata cgaagttatt accgggtagg      60

ggaggcgctt                                                             70


<210>  4
<211>  41
<212>  DNA
<213>  Artificial Sequence

<220>
<223>  Primer

<400>  4
ggggatccac tagttctaga cgaaaggccc ggagatgagg a                          41


<210>  5
<211>  42
<212>  DNA
<213>  Artificial Sequence

<220>
<223>  Primer

<400>  5
ctccgggcct ttcgtgccac catgaaaaag cctgaactca cc                         42


<210>  6
<211>  55
<212>  DNA
<213>  Artificial Sequence

<220>
<223>  Primer

<400>  6
ataacttcgt ataatgtatg ctatacgaag ttatcaacta tgaaacatta tcata           55


<210>  7
<211>  38
<212>  DNA
<213>  Artificial Sequence

<220>
<223>  Primer

<400>  7
attatacgaa gttatccacc gagtacaagc ccacggtg                              38


<210>  8
<211>  36
<212>  DNA
<213>  Artificial Sequence

<220>
<223>  Primer

<400>  8
ggggatccac tagttgaagt tcctatactt tctaga                                36


<210>  9
<211>  43
<212>  DNA
<213>  Artificial Sequence

<220>
<223>  Primer

<400>  9
ctccaccgcg gtggcggccg ccttcaaaag ctccttctgt tgt                        43

<210>  10
<211>  38
<212>  DNA
<213>  Artificial Sequence

<220>
<223>  Primer

<400>  10
atgatgcggc cgctagcctt ggaaaagaca aaggcagt                              38


<210>  11
<211>  43
<212>  DNA
<213>  Artificial Sequence

<220>
<223>  Primer

<400>  11
aggaattcga tatcatttaa atgaggggga ctagcaggga gga                        43


<210>  12
<211>  42
<212>  DNA
<213>  Artificial Sequence

<220>
<223>  Primer

<400>  12
tcgacggtat cgatatttaa atccccgaaa gtcaggacgt tg                        42


<210>  13
<211>  34
<212>  DNA
<213>  Artificial Sequence

<220>
<223>  Primer

<400>  13
gctagcggcc gcatcatgag accatctgta cctg                                  34


<210>  14
<211>  41
<212>  DNA
<213>  Artificial Sequence

<220>
<223>  Primer

<400>  14
atacgaagtt atagctagtc ttccgtgatg gcctcacacc a                          41


<210>  15

<211>  24
<212>  DNA
<213>  Artificial Sequence

<220>
<223>  Sequence for Crispr/Cas9

<400>  15
caccgaggtt agtctgactg tgtg                                              24


<210>  16
<211>  24
<212>  DNA
<213>  Artificial Sequence

<220>
<223>  Sequence for Crispr/Cas9

<400>  16
aaaccacaca gtcagactaa cctc                                              24


<210>  17
<211>  24
<212>  DNA
<213>  Artificial Sequence

<220>
<223>  Sequence for Crispr/Cas9

<400>  17
caccctgccg ctgcccagtg gttg                                              24


<210>  18
<211>  24
<212>  DNA
<213>  Artificial Sequence

<220>
<223>  Sequence for Crispr/Cas9

<400>  18
aaaccaacca ctgggcagcg gcag                                              24


<210>  19
<211>  24
<212>  DNA
<213>  Artificial Sequence

<220>
<223>  Sequence for Crispr/Cas9

<400>  19
caccgagaat caaaatcggt gaat                                              24


<210>  20
<211>  24
<212>  DNA
<213>  Artificial Sequence

<220>
<223>  Sequence for Crispr/Cas9

<400>  20
aaacattcac cgattttgat tctc                                              24


<210>  21
<211>  20
<212>  DNA
<213>  Artificial Sequence

<220>
<223>  Primer

<400>  21
ccttgtccat cactggcatc                                                   20


<210>  22
<211>  21
<212>  DNA
<213>  Artificial Sequence

<220>
<223>  Primer

<400>  22
aaagtcagat ttgttgctcc a                                                 21


<210>  23
<211>  45
<212>  DNA
<213>  Artificial Sequence

<220>
<223>  Primer

<400>  23
aaggaaccaa ttcagtcgac caccatgctg ctgcttctgc tgctt                       45


<210>  24
<211>  75
<212>  DNA
<213>  Artificial Sequence

<220>
<223>  Primer

<400>  24
ctcctccacg tctccagcct gcttcagcag gctgaagtta gtagctccgc ttccgcctct      60

ggaatccttt ctctt                                                        75


<210>  25
<211>  49
<212>  DNA
<213>  Artificial Sequence

<220>
<223>  Primer

<400>  25
tggagacgtg gaggagaacc ctggacctat gatgatatcc ttgagagtt                49


<210>  26
<211>  41
<212>  DNA
<213>  Artificial Sequence

<220>
<223>  Primer

<400>  26
tcgagtgcgg ccgcgaattc tcagctggac cacagccgca g                        41


<210>  27
<211>  40
<212>  DNA
<213>  Artificial Sequence

<220>
<223>  Primer

<400>  27
tatacgaagt tatcgctagc ggtttgccgc cagaacacag                          40


<210>  28
<211>  40
<212>  DNA
<213>  Artificial Sequence

<220>
<223>  Primer

<400>  28
tacaaacttg tgatgctagc gtagttttca cgacacctga                          40


<210>  29
<211>  40
<212>  DNA
<213>  Artificial Sequence

<220>
<223>  Primer

<400>  29
tatacgaagt tatcgctagc gccccggctc tgactgaccg                          40


<210>  30
<211>  40
<212>  DNA
<213>  Artificial Sequence

<220>
<223>  Primer

<400>  30
tacaaacttg tgatgctagc gacagcacaa taaccagcac                              40


<210>  31
<211>  24
<212>  DNA
<213>  Artificial Sequence

<220>
<223>  Sequence for Crispr/Cas9

<400>  31
caccctgccg ctgcccagtg gttg                                               24


<210>  32
<211>  24
<212>  DNA
<213>  Artificial Sequence

<220>
<223>  Sequence for Crispr/Cas9

<400>  32
aaaccaacca ctgggcagcg gcag                                               24


<210>  33
<211>  22
<212>  DNA
<213>  Artificial Sequence

<220>
<223>  Primer

<400>  33
acggctgaaa tctccctaac cc                                                 22


<210>  34
<211>  22
<212>  DNA
<213>  Artificial Sequence

<220>
<223>  Primer

<400>  34
tacttccatt tgtcacgtcc tg                                                 22


<210>  35
<211>  19
<212>  DNA
<213>  Artificial Sequence

<220>
<223>  Primer

<400>  35
cctgctgcaa cttacctcc                                                     19

<210>  36
<211>  20
<212>  DNA
<213>  Artificial Sequence

<220>
<223>  Primer

<400>  36
ggggaccgag gggctggaag                                                    20


<210>  37
<211>  24
<212>  DNA
<213>  Artificial Sequence

<220>
<223>  Sequence for Crispr/Cas9n

<400>  37
caccggtaga aggaggctta tacc                                               24


<210>  38
<211>  24
<212>  DNA
<213>  Artificial Sequence

<220>
<223>  Sequence for Crispr/Cas9n

<400>  38
aaacggtata agcctccttc tacc                                               24


<210>  39
<211>  24
<212>  DNA
<213>  Artificial Sequence

<220>
<223>  Sequence for Crispr/Cas9n

<400>  39
caccgggtgg gcatgtgcgt gtgt                                               24


<210>  40
<211>  24
<212>  DNA
<213>  Artificial Sequence

<220>
<223>  Sequence for Crispr/Cas9n

<400>  40
aaacacacac gcacatgccc accc                                               24


<210>  41
<211>  25

<212>  DNA
<213>  Artificial Sequence

<220>
<223>  Sequence for Crispr/Cas9n

<400>  41
caccgcaaac acagcgacct cgggt                                          25


<210>  42
<211>  25
<212>  DNA
<213>  Artificial Sequence

<220>
<223>  Sequence for Crispr/Cas9n

<400>  42
aaacacccga ggtcgctgtg tttgc                                          25


<210>  43
<211>  24
<212>  DNA
<213>  Artificial Sequence

<220>
<223>  Sequence for Crispr/Cas9n

<400>  43
caccagagat ctcccacacc caaa                                           24


<210>  44
<211>  24
<212>  DNA
<213>  Artificial Sequence

<220>
<223>  Sequence for Crispr/Cas9n

<400>  44
aaactttggg tgtgggagat ctct                                           24


<210>  45
<211>  21
<212>  DNA
<213>  Artificial Sequence

<220>
<223>  Primer

<400>  45
gtcatgggca aagattacca c                                              21


<210>  46
<211>  21
<212>  DNA
<213>  Artificial Sequence

<220>
<223>  Primer

<400>  46
ggtagctggt ctcacctaat c                                                21


<210>  47
<211>  20
<212>  DNA
<213>  Artificial Sequence

<220>
<223>  Primer

<400>  47
ggcagctaca tccctacctt                                                  20


<210>  48
<211>  21
<212>  DNA
<213>  Artificial Sequence

<220>
<223>  Primer

<400>  48
ccactttgct gtcttggcct t                                                21


<210>  49
<211>  21
<212>  DNA
<213>  Artificial Sequence

<220>
<223>  Primer

<400>  49
aatcatcgtg ccctcccgct a                                                21


<210>  50
<211>  20
<212>  DNA
<213>  Artificial Sequence

<220>
<223>  Primer

<400>  50
tcagccacct acctctgtgt                                                  20


<210>  51
<211>  65
<212>  DNA
<213>  Artificial Sequence

<220>
<223>  Vbeta20-1 and Cbeta2 regions of clone #10

<400> 51
atgctgctgc ttctgctgct tctggggcca ggtataagcc tccttcgggt gggcctggcc 60

acagg 65

## Description

### [Technical Field]

This disclosure relates to cells into which an exogenous T cell receptor or Chimeric Antigen Receptor is introduced.

### [Background Art]

Therapeutic procedures by using mature T cells transduced by introducing genes encoding an antigen specific receptor such as a disease-specific T cell receptor (TCR) and Chimeric Antigen Receptor (CAR) have been proposed. In particular, therapeutic method using CAR-T cells or the cells into which CAR is introduced has already been approved and clinically used. CAR-T therapy is a so-called autologous transplant system in which T cells derived from the patient are used to introduce genes encoding the CAR. Genes are randomly introduced in the genome by means of a retrovirus or lentivirus. When genes are introduced by such a method, there is a risk of damaging normal genes and activating cancer genes. On the other hand, a method of knocking genes encoding CAR in a re-arranged TCR gene locus by means of a genome editing method has been proposed (Nature, 543:113, 2017). In this method, the introduced genes are under the physiological TCR expression control system and the CAR-T cells produced in this way are deemed to have higher functionality.

The present inventors had proposed a method to introduce a TCR in pluripotent stem cells (Patent Literature 1). The pluripotent stem cells introduced with the TCR gene are then differentiated into T cells and used for cell therapy. M Sadelain proposed to introduce a CAR gene to pluripotent stem cell (Patent Literature 2 and Non-patent literature 1).

To date, there is no report in which genes encoding either TCR or CAR are introduced in a TCR locus of a cell that is not a T cell. The TCR gene loci in a cell other than T cell will not be rearranged. There is no report regarding knocking a TCR or CAR gene in a TCR locus of a cell where no genetic rearrangement has occurred.

Recombinase-mediated cassette exchange (RMCE) has been known as a procedure for introducing genes into cells. In the procedure, material cells having a structure like "cassette deck" may be used. The cell with "cassette deck structure" has a specific site in its genome which can be exchanged with a gene of interest or "cassette tape gene". The exogenous gene (gene of interest) is introduced in the host cells by using a recombinase such as Cre or Flippase, like exchanging cassette tapes. This procedure has been applied to manipulate a T cell line (non-patent literature 2). There is no report regarding application of the RMCE to a T cell receptor gene locus.

In general, TCR or CAR genes have been introduced into mature T cells. Some of the present inventors proposed introducing TCR genes into pluripotent stem cells (Patent literatures 3-5). Introduction of CAR genes into pluripotent stem cells has also been proposed. In those procedures, TCR or CAR genes are randomly introduced in the genome of the cells by using, for example, lentivirus vectors. However, it is better to knock-in the TCR or CAR gene in an original TCR locus of the host cell for expecting the physiological expression pattern of the introduced TCR or CAR gene. Actually, there is a report in which a CAR gene was introduced in a rearranged TCR locus in a mature T cell. In general, all types of cells have TCR loci. However, TCR gene rearrangement does not occur in the cells other than T cells. An exogenous TCR/CAR cannot be expressed in a cell other than T cell just by inserting TCR/CAR gene in a TCR locus of the cell. Although various types of TCR/CAR genes are expected to be used in cell therapies, knocking in every single TCR/CAR to a desired locus in the host cell genome will require a lot of time and cost.

### [CITATION LIST]

### [PATENT LITERATURE]

[PTL 1] WO2016/010154
[PTL 2] WO2014/165707
[PTL 3] WO2016/010153
[PTL 4] WO2016/010154
[PTL 5] WO2016/010155

### [NON PATENT LITERATURE]

[NPL 1] Themeli et al., Nat Biotechnol. (2013) 928-33
[NPL 2] Gong Ying et al., Journal of Cell Biology (2015) 1481-1489

### SUMMARY OF THE INVENTION

### PROBLEMS TO BE SOLVED BY THE INVENTION

An object of the present application to provide an efficient method to provide cells stably expressing exogenous TCR or CAR genes that can be used for cell therapy. Another object of the present application is to provide cells bearing empty "cassette deck that can be used for introducing a cassette of a TCR or CAR gene by means of RMCE.

### MEANS FOR SOLVING THE PROBLEM

The present application provide a method for producing cells incorporating an antigen specific receptor gene, which comprises the step of introducing an exogenous TCR or CAR gene into material cells so that the introduced gene is expressed under the T cell receptor expression control system of the material cells.

In one aspect, the present application provides a method for producing cells into which a gene encoding an antigen specific receptor is introduced, comprising the step of introducing a gene including an exogenous TCR or CAR gene into a material cell so that the exogenous gene is introduced between a C region enhancer and a V region promoter of a TCR locus so that the C region enhancer and the V region promoter are sufficiently close each other to exert the TCR expression control system to express the intervening gene.

In another aspect, the present application provides a method for producing a cell into which a gene encoding an antigen specific receptor is introduced, comprising the step of introducing genes in order from upstream to downstream, an exogenous V region promoter gene and genes including exogenous TCR or CAR gene, into upstream of a C region enhancer in a TCR gene locus in a material cell so that the introduced V region promoter and the C region enhancer are sufficiently close each other to exert the TCR expression control system to express the intervening gene.

In yet another aspect, the present application provides a method for producing a cell into which a gene encoding an antigen specific receptor is introduced, comprising the step of introducing genes comprising, in order from upstream to downstream, an exogenous TCR or CAR gene and an exogenous C region enhancer into upstream of a V region promoter in a TCR locus of a material cell, so that the V region promoter and the introduced C region enhancer are sufficiently close each other to exert the TCR expression control system to express the intervening gene.

The present application also provides a material cell for antigen receptor transduction that includes in a TCR locus in the material cell genome, in order from upstream to downstream, a V region promoter, a first drug resistance gene sandwitched by first recombinase target sequences, a second drug resistance gene and a second recombinase target sequence, and a C region enhancer.

The present application further provides a method for producing cells for cell therapy, comprising the step of inducing differentiation of material cells in which an exogenous antigen receptor gene has been introduced into a TCR locus by the method of the present application into T cells.

### EFFECT OF THE INVENTION

The method of the present application makes it possible to produce highly versatile cells that can be used for cell therapy using cells expressing TCRs and CARs. In addition, an exogenous TCR gene or CAR gene can be easily introduced into the material cells for antigen receptor transduction, and stable expression of TCR/CAR gene is guaranteed in the final product. As a result, immunotherapeutic cells expressing exogenous TCRs or CARs can be efficiently and easily generated.

### BRIEF EXPLANATION OF THE DRAWINGS

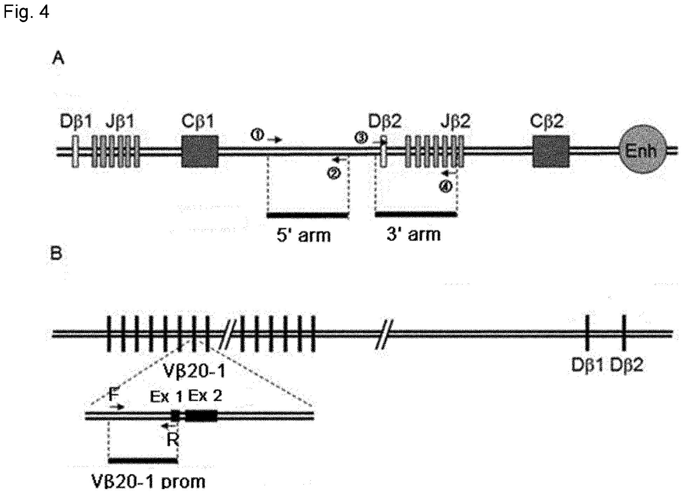

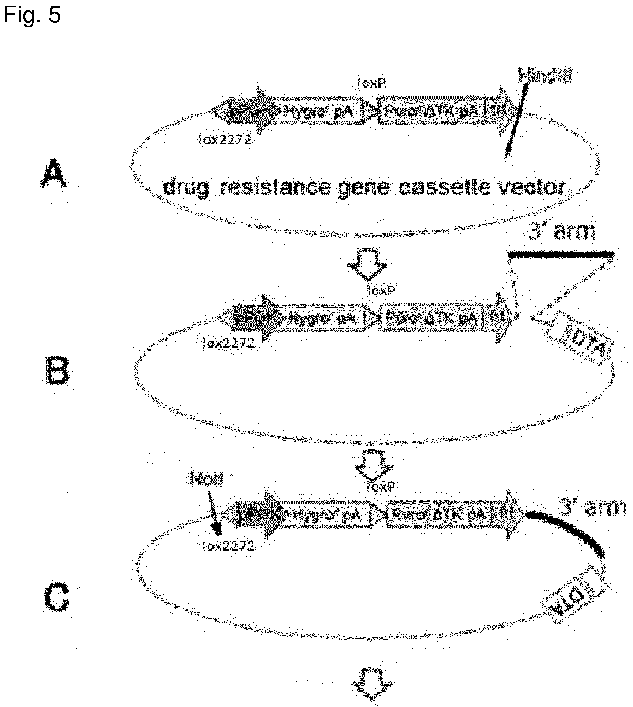

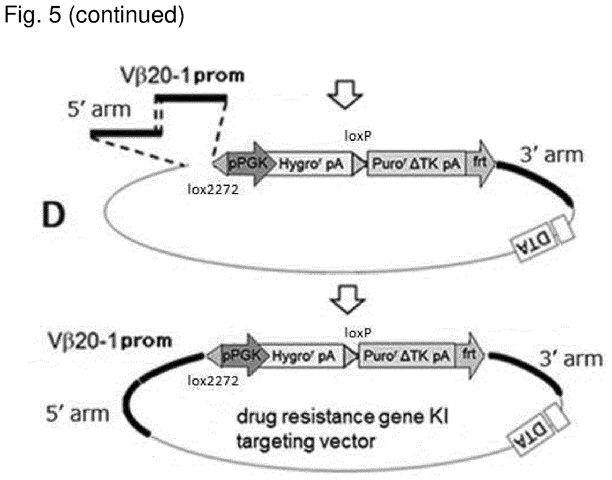

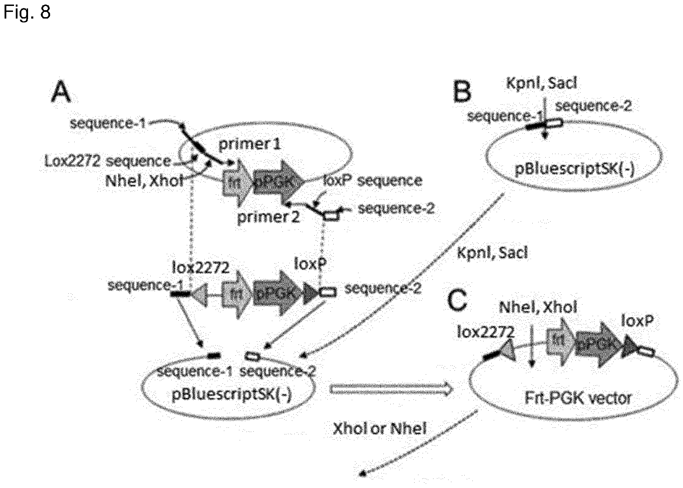

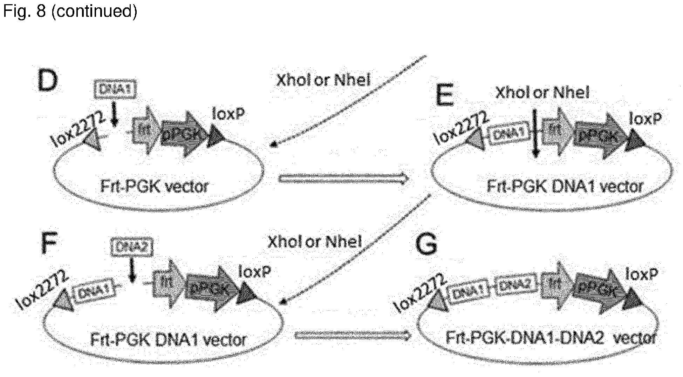

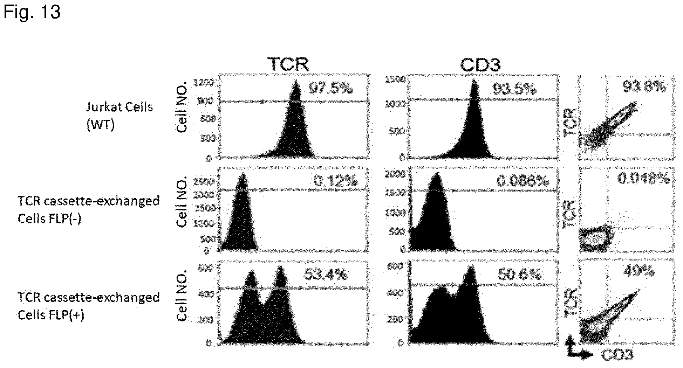

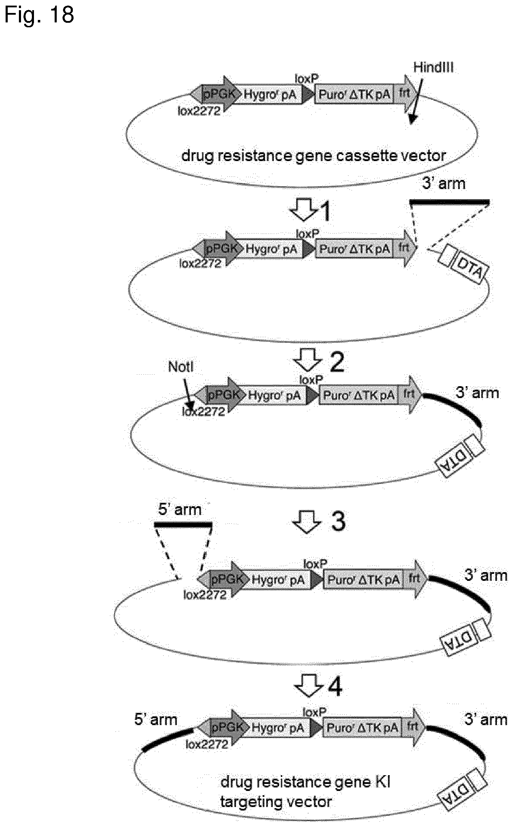

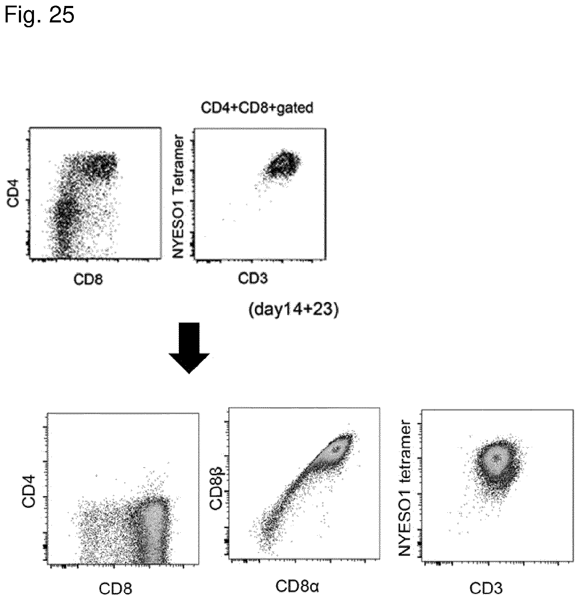

[Fig. 1]
   A schematic diagram of TCRαβ and a schematic diagram of the genetic rearrangement of the TCRβ locus are shown. In the figure, P indicates a promoter and E indicates an enhancer. In V-DJ rearrangement, recombination brings the enhancer and promoter closer together and the TCR is expressed.
[Fig. 2]
   Schematic diagrams showing the concept of the method of this application. In this diagrams, P represents the promoter and E represents the enhancer.
[Fig. 3-1]
   A schematic diagram showing the procedure for constructing the drug resistance gene cassette vector of the example. A pBRBlII-AscI_FRTPGKpacΔtkpA_AscI vector carrying the promoter of the mouse phosphoglycerate kinase (PGK) gene (pPGK) was used. Primer 1 and Primer 2 in (A) have the same sequences as sequence-1 and sequence-2, which are the DNA sequences on the 5' side and 3' side of the restriction enzyme cleavage site of the pBRMC1DTApA vector in (B), respectively. The resulting PCR product has sequences identical to sequence-1 and sequence-2 in the pBRMC1DTApA vector at both ends. By the Gibson assembly method, the vector and the PCR product are combined through the same sequences to form the structure in (C) .
[Fig. 3-2]
   From the PB-flox(CAG-mCherry-IH;TRE3G-miR-155-LacZa) vector of (E) and the pBRBlII-AscI_FRTPGKpacΔtkpA_AscI vector of (F), respectively, PCR products with sequence-3 and sequence-4 portions at the PGK vector (D) restriction enzyme cleavage site were obtained. The Gibson assembly method was used to link the DNA fragments together through the same sequences to obtain a drug resistance gene cassette vector of (G). After the fragments were linked to the vector, it was confirmed that the vector contained such sequences.
[Fig. 4]
   A schematic diagram showing the procedure for obtaining DNA fragments of 5' and 3' arms and a promoter for the construction of a drug resistance gene cassette knock-in targeting vector. Primers including Primer 5'-1(1) and Primer 3'-1(2), as well as Primer5'-2(3) and Primer3'-2(4) were designed based on the DNA sequence of the Dβ2 region of the human TCRβ locus(A), and the DNA fragments used as the 5'arm and 3'arm (Figure 5) for constructing the drug resistance gene knock-in targeting vector were obtained by PCR. Primers (F and R) were designed based on the DNA sequence of a V region of the human TCRβ locus (B, left side), and the Vβ20-1 promoter DNA fragment was obtained in the same way. After the fragments were linked to the vector, it was confirmed that the vector contained such fragments and was used in subsequent procedures.
[Fig. 5]
   A schematic diagram showing the procedure for constructing a targeting vector for knocking-in the drug resistance gene into the Dβ2 region in a TCRβ locus. The drug resistance cassette vector (A) was cleaved with restriction enzymes and the 3' arm DNA fragment (Figure 4A) was introduced into the cleavage site by Gibson assembly method (B). Similarly, the 5' arm DNA fragment and the Vβ20-1 promoter DNA fragment were introduced (C,D) .
[Fig. 6]
   A schematic diagram showing the procedure to knock-in a drug resistance gene cassette into the Dβ2 region of the Jurkat cell TCRβ locus by homologous recombination. This diagram shows the DJ region of the human TCRβ locus (upper panel), the drug resistance gene knock-in targeting vector (KI targeting vector) (middle panel), and the DJ region of the TCRβ locus after homologous recombination (lower panel). CRISPR/Cas9n cuts one strand of each of the two strands of DNA (nick). Nick promotes homologous recombination. In homologous recombination, the 5' and 3' arm portions of the KI targeting vector, along with the portions flanked by both arms, are replaced with the corresponding 5' and 3' arm portions in the TCRβlocus. As a result, only a part of the KI targeting vector sandwiched between the 5' and 3' arms is introduced into the genomic DNA of the material cell (lower panel).
[Fig. 7]
   A schematic diagram showing the procedure to construct the TCRβ-p2A-TCRα vector (TCR donor vector). Approximately 15bp on the 5' side of the restriction enzyme cleavage site of the pENTR1A vector is designated sequence-1 and approximately 15bp on the 3' side is designated sequence-2 (A). Primer 1(B) has a part of the sequence of the TCRβ and a sequence identical to sequence-1, and Primer 4(C) has a part of TCRα and a sequence identical to sequence-2. Primer 2(B) and primer 3(C) have a part of the TCRβ sequence and most of the p2A sequence, and a part of the TCRα sequence and part of the p2A sequence, respectively. There is a 16bp overlap between the p2A sequences (D). The vector and the PCR product were linked through the overlap with each other by Gibson assembly method to obtain the TCR donor vector (E). The sequences of the DNA fragments obtained by PCR were confirmed after linking to the vector. Primer 2 and primer 4 can be used to amplify all human TCRβ and TCRα, respectively. AttL1 and attL2 were used for producing the TCR cassette exchange vector (FIG. 8).
[Fig. 8]
   A schematic diagram of the procedure for making the vector backbone in the construction of the cassette exchange vector. PCR reactions were performed with primer 1 and primer 2 using the pBRBlII-AscI_FRTPGKpacΔtkpA_AscI plasmid vector, in which the Frt and PGK promoters are located consecutively, as the template (A). Primer 1(A) has a part of the frt, restriction enzyme , lox2272, and a sequence identical to the sequence-1 on the pBluescriptSK(-) vector of (B), and primer 2 has a part of the PGK promoter, loxP, and a sequence identical to the sequence-2 on the pBluescriptSK(-) vector of (B). The obtained PCR products were linked to the restriction enzyme-cleaved vector (B) by the Gibson assembly method to give Frt-PGK vector as in FIG. 3(C). The Frt-PGK vector of (C) was cleaved at the restriction enzyme recognition site inside primer 1, and DNA 1 having a sequence complementary to the cleaved end was linked to it using the DNA ligation enzyme (E). The vector could be cleaved at the restriction enzyme recognition site designed in primer 1 again, and DNA 2 can be linked to it in the same way as DNA 1. By repeating this process multiple times, multiple arbitrary DNA fragments could be introduced between the lox2272 and frt sequences. The introduction can be done either by DNA linkage reaction or by Gibson assembly (D-G).
[Fig. 9]
   A schematic diagram showing the procedure for constructing a pre-cassette exchange vector for the construction of a cassette exchange vector. The RfA cassette containing the attR1-ccdb-attR2 DNA was obtained by digesting the CSIV-TRE-RfA-CMV-KT vector with restriction enzymes NheI and XhoI. The obtained fragment was linked to the Ftr-PGK vector digested with restriction enzymes NheI and XhoI, in the manner of FIG. 8D to 8G (B). Pre-cassette exchange vectors 1 to 3 (B, D, F) were constructed based on the Frt-PGK vector (A) in the manner shown in FIG. 8D to 8G, in which the attR1-ccdb-attR2 DNA fragment (B), attR1-ccdb-attR2 DNA fragment and poly A addition sequence (D), attR1-ccdb-attR2 DNA fragment, poly A addition sequence and intron (between lox2272 and attR1) (F) were introduced. The poly-A addition sequence was obtained from the plasmid vector using restriction enzymes (C). The intron sequences were obtained by PCR to have a part of exon without translation initiation codons (E). The sequences of the DNA fragments obtained by PCR were confirmed after linking to the vector.
[Fig. 10]
   A schematic diagram of the procedure for producing a TCR cassette exchange vector. The top panel shows a pre-cassette exchange vector 3, the middle panel shows a TCR donor vector, and the bottom panel shows the TCR cassette exchange vector. In the pre-cassette exchange vector and the TCR donor vector, the attR1 sequence recombined with the attL1 sequence and the attR2 sequence recombined with the attL2 sequence, and the portion flanked by each was replaced (bottom row). The attR1 (2) sequence recombined with the attL1 (2) sequence to form the attB1 (2) sequence.
[Fig. 11]
   A schematic diagram of the procedure to exchange the drug resistance gene cassette with the TCR cassette (generation of TCR cassette exchanged Jurkat cells). Upper panel shows the DJ region in the human TCRβ locus after homologous recombination with the drug resistance gene KI targeting vector. Middle panel shows the TCR cassette exchange vector. The bottom panel shows the Dβ2 region of the human TCRβ locus after the cassette exchange. When the TCR cassette exchange vector and a Cre recombinase expression vector are introduced together into drug resistance gene KI-Jurkat cells, Cre recombinase promotes recombination between lox2272 and lox2272, and between loxP and loxP. As a result, the part flanked by lox2272 and loxP sequences is replaced (cassette exchange). After the cassette exchange, expression of the puromycin resistance gene is initiated.
[Fig. 12]
   A schematic diagram of the procedure to generate Jurkat cells expressing an exogenous TCR. FLP is expressed in the cells after the TCR cassette exchange. The portion flanked by frt sequences is deleted by the action of FLP. As a result, the enhancer (Enh) efficiently acts on the promoter (Vβ20-1 promoter) and the downstream gene (TCR) is expressed.
[Fig. 13]
   FACS analysis to examine the expression of exogenously introduced TCR and CD3 on the cell surface in the cassette exchanged Jurkat cells. Wild Type (WT) Jurkat cells expressed endogenous TCR (upper panel). Exogenously introduced TCR was not expressed in the cassette exchanged Jurkat cells without FLP expression (middle panel). In contrast, the Jurkat cells expressed exogenously introduced TCR upon FLP expression but a high percentage of the population did not express the TCR (bottom panel) .
[Fig. 14]
   A schematic diagram showing the procedure to remove the cells in which FLP-mediated recombinantion is failed with ganciclovir. Ganciclovir does not affect cells that have been successfully recombined and lost puro^{r}-Δtk gene by FLP (A). In the cells in which FLP recombination fails, ganciclovir is phosphorylated by purorΔTK-and the phosphorylated ganciclovir inhibits DNA replication. As a result, cells that failed FLP-mediated recombination cannot replicate DNA in the presence of ganciclovir and are removed from the cell population by means of cell death(B).
[Fig. 15]
   FACS analysis of the expression levels of TCR and CD3 on the cell surface. Endogenous TCR expression in WT Jurkat cells (upper panel) . Cells without FLP expression did not express TCR (middle row). Exogenously introduced TCR expression was observed in the FLP-expressing cells (bottom row), and ganciclovir selection greatly reduced proportion of the non-TCR expressing cells compared to FIG. 13.
[Fig. 16]
   (A) The site in the human TCRβ region that are cleaved by CRISPR/Cas9 and the sites corresponding to each primer are shown. (B) A schematic diagram of the drug resistance gene targeting vector and the sites corresponding to the respective primers. (C) Electrophoretic photograph of the PCR products obtained with the primers to confirm the drug resistance gene cassette knock-in.
[Fig. 17]
   A schematic diagram showing the procedure to obtain 5' and 3' arms and a DNA fragment of a promoter for the construction of targeting vectors for drug resistance gene cassette knock-in. The primers were designed based on the DNA sequences of the Vβ (A) and Cβ2 regions in the human TCRβ locus, and the DNA fragments used as the 5' and 3' arms (FIG. 18) for constructing the drug resistance gene knock-in targeting vector are obtained by PCR. The sequence of the DNA fragment obtained by PCR is confirmed after linking to the vector and used in the subsequent procedures.
[Fig. 18]
   A schematic diagram showing the procedure for constructing a drug resistance gene knock-in targeting vector that was used for knocking-in the drug resistance gene cassette into the region where the Vβ20-1 and Cβ2 genes in the TCRβ locus were linked. The drug-resistance gene cassette vector was cleaved with restriction enzymes and the 3' arm DNA fragment was introduced into the cleavage site using the Gibson assembly method. Similarly, a 5' arm DNA fragment was introduced. As a result, drug resistance gene knock-in targeting vector was obtained.
[Fig. 19]
   A schematic diagram showing the procedure for knocking-in the drug resistance gene cassette by homologous recombination into the site where the Vβ20-1 and Cβ2 genes in the TCRβ locus are linked in the Jurkat cell (upper panel). The drug resistance gene knock-in targeting vector (KI targeting vector) (middle panel). The TCRβ gene after homologous recombination (lower panel). In the homologous recombination, the 5' and 3' arms of the KI targeting vector, along with the sequence flanked by both arms, can be replaced with the corresponding 5' and 3' arms in the TCRβ locus, respectively. As a result, only the sequence of the KI targeting vector sandwiched between the 5' and 3' arms can be introduced into the genomic DNA of the material cell (lower panel).
[Fig. 20]
   A schematic diagram of the procedure of Example 3, in which the region between the Vβ20-1 and Cβ2 genes was removed from the human TCRβ locus where genetic rearrangement had not occurred. The top panel shows the TCRβ locus and the CRISPR/Cas9n target sites. Each two target sites were provided for the Vβ20-1 and Cβ2 genes, respectively (vertical lines). The bottom panel shows the TCRβ locus and the linkage site after the intergenic region was removed. The vertical line indicates the linkage sites of the Vβ20-1 and Cβ2 genes.
[Fig. 21]
   The results of Example 3 are shown. A cell line wherein a region of approximately 180kbp between the Vβ20-1 and Cβ2 genes was removed from its genome was isolated.

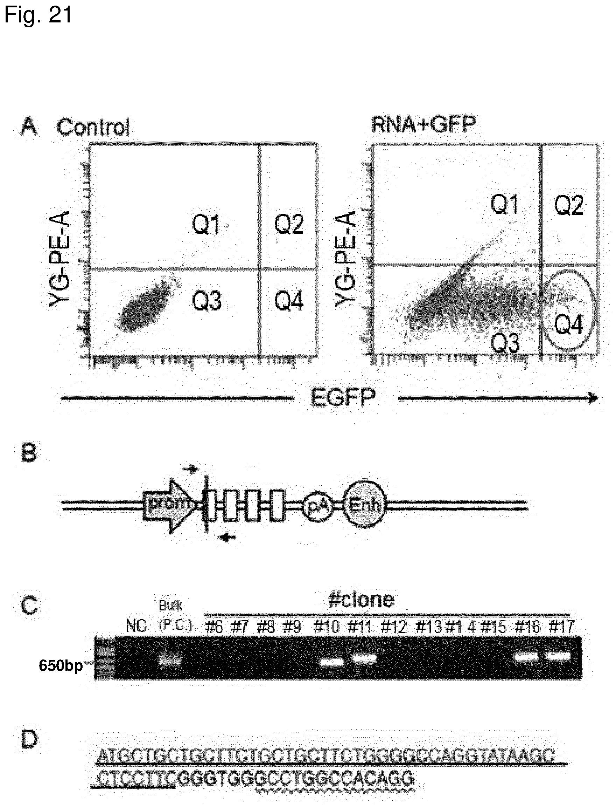

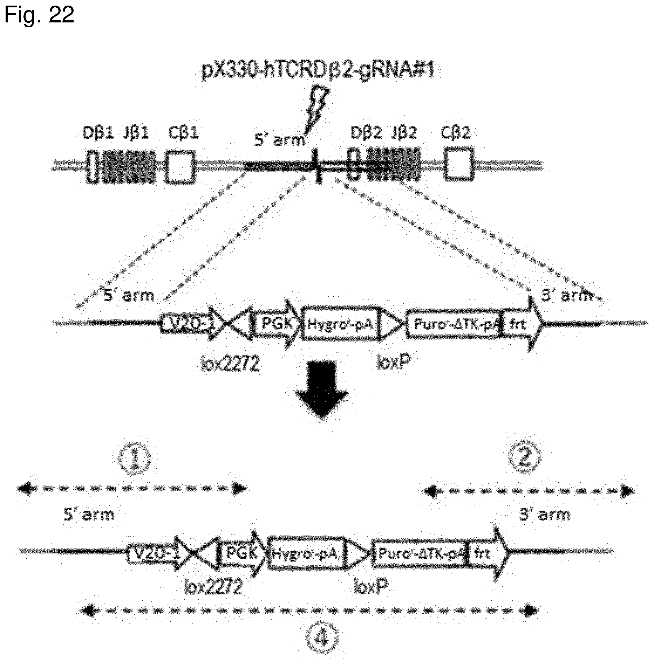

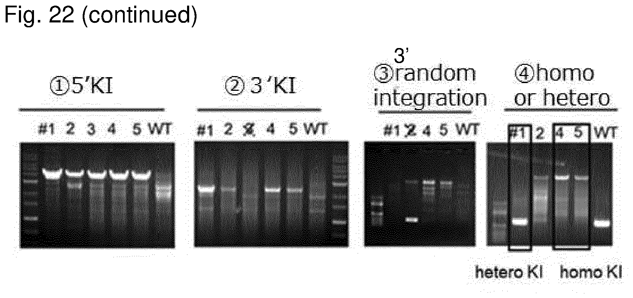

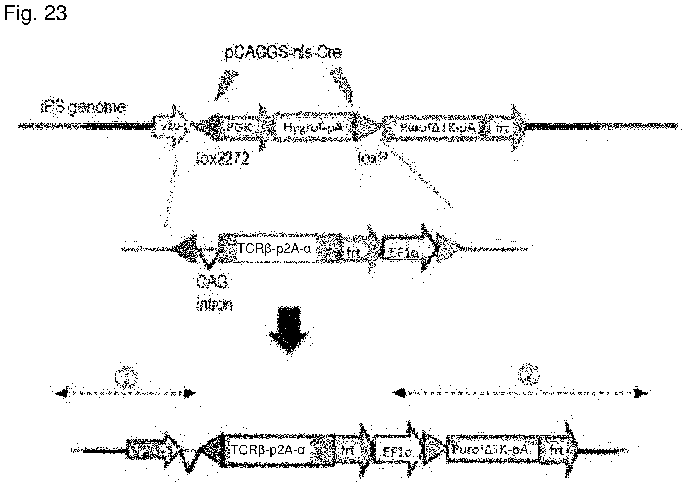

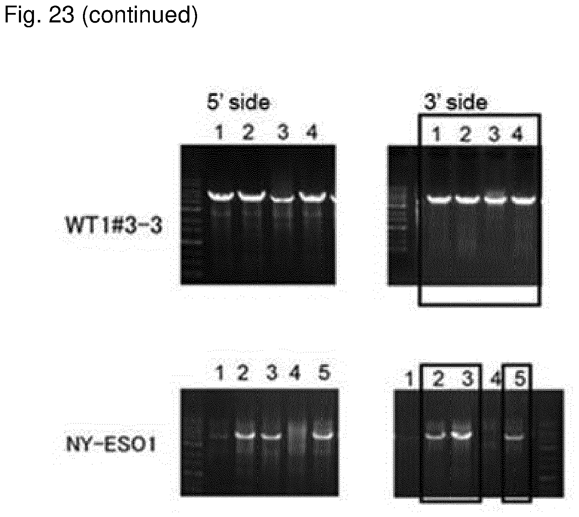

   Fig. 21A shows FACS analysis of the cells that were not transfected (left panel) and were transfected (right panel). The cells with high expression of EGFP were isolated by sorting (circled area) from the transfected cells.
   Figure 21B represents the TCRβ locus after the 180kbp region had been removed. Arrows indicate forward and reverse primers. Vertical line indicates the linkage site between the genes after the removal.
   FIG. 21C is electrophoretic result of PCR products obtained using template DNAs from the genomic DNA of each clonal cell.
   Figure 21D is a part of the PCR product of clone #10 (sequence number 51). The region that was about 180 kbp became 6 bp. Underlined (straight line) part: Vβ20-1 side array, underlined (wavy line) part: Cβ2 side array. There was originally about 180kbp between the two, but it became 6 bp.
[Fig. 22]
   A schematic diagram of the example in which the drug resistance cassette deck was knocked into the Dβ2 region in the TCRβ locus of a human iPS cell.
[Fig. 23]
   A schematic diagram of the example in which the "cassette tape" was exchanged in the iPS cells.
[Fig. 24]
   A schematic diagram of the example in which PurorΔTK region in the cassette-exchanged TCR-iPS cells was removed.
[Fig. 25]
   Results showing that the CTLs were obtained from the cassette-exchanged TCR-KI-iPS cells by inducing differentiation of the cells into T cells.
[Fig. 26]
   Results showing that the CTLs in Figure 25 had antigen-specific cytotoxic activity.
[Fig. 27]
   A schematic diagram of the human TCRβ gene DJ region in a Jurkat-TCR1 cell.
[Fig. 28]
   A schematic diagram showing the exchange of the TCR cassette tape by Cre recombinase. Top panel shows the TCRβ locus DJ region into which TCR1 is incorporated. The middle panel shows the TCR2 cassette tape exchange vector. The bottom panel shows the human TCRβ locus Dβ2 region after the cassette tape exchange. When the TCR cassette exchange vector and Cre recombinase expression vector are introduced together into Jurkat-TCR1 cells, Cre recombinase mediates recombination between lox2272s and between loxPs. This results in the replacement of the part sandwiched between lox2272 and loxP (cassette tape exchange). In the cells where cassette tape exchange has occurred, TCR1 is replaced by TCR2 (Jurkat-TCR 2) .
[Fig. 29]
   A: A schematic diagram of the TCRβ locus Dβ2 region in which TCR1 (upper panel) or TCR2 (lower panel) gene is incorporated. Positions of primers used in the PCR reaction are indicated.
   B: Electrophoretic analysis of PCR products in each primer combination. PCR 1 shows the results of PCR reactions using the genomic DNA of TCR2-introduced Jurkat cells, PCR 2 shows the results of PCR reactions using Jurkat-TCR1 cells, and PCR 3 shows the results of PCR reactions using the genomic DNA of Jurkat-TCR1 cells transfected with the TCR2 cassette exchange vector and Cre recombinase expression vector.

### [Embodiments]

In this specification and claims, when a numerical value is accompanied by the term "about", it is intended to include a range within ±10% of that value. For example, "approximately 20" shall include "18 to 22". The range of numbers includes all numbers between the two endpoints and the numbers at both endpoints. The "about" for a range applies to both endpoints of that range. Thus, for example, "about 20 to 30" shall include "18 to 33".

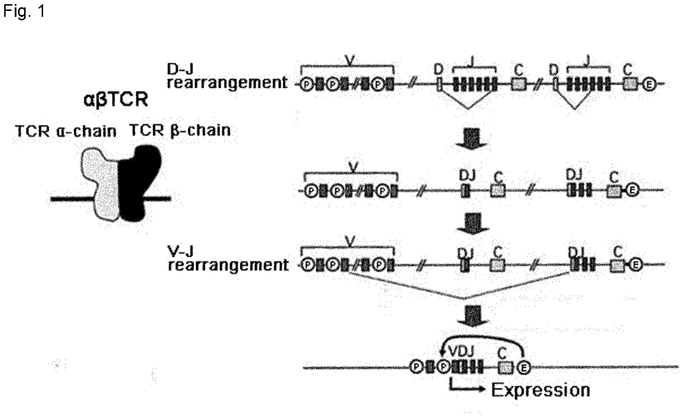

A TCR gene is expressed when a promoter upstream of the V region (V region promoter) and an enhancer downstream of the C region (C region enhancer) become close each other due to genetic rearrangement. A schematic diagram of the mechanism of TCRβ gene rearrangement and expression is provided as Fig. 1.

If the rearranged TCR gene of a T cell is replaced with an exogenous rearranged TCR or CAR gene (hereinafter collectively referred to as TCR/CAR gene), the transduced TCR/CAR gene is expressed under the TCR expression control system. If the TCR/CAR gene is simply introduced into a TCR locus of the genome of a cell that has not undergone genetic rearrangement, the original TCR expression control system will not work. TCR expression control system is only activated when the V region promoter and the C region enhancer become close to each other. In other words, the present application provides a method of expressing an exogenous antigen receptor gene into a material cell by knocking an exogenous TCR/CAR gene in such a way as to bring the V region promoter and C region enhancer in a TCR locus into close each other.

In the present application, "antigen receptor gene" means a TCR gene or a CAR gene. The exogenous TCR gene or exogenous CAR gene is not particularly limited, and may be selected from those known as rearranged TCR genes or CAR genes. Alternatively, the TCR gene may be amplified by known methods from T cells specific for the target antigen of the cell therapy and used, or the CAR gene for the target antigen may be constructed.

The term "Chimeric Antigen Receptor" or "CAR" refers to a recombinant polypeptide construct comprising at least an extracellular antigen binding domain, a transmembrane domain, a cytoplasmic signaling domain including a cytoplasmic sequence of CD3ζ chain sufficient to stimulate T cells when bound to an antigen, and optionally one or more (for example, 2, 3 or 4) cytoplasmic costimulatory proteins that co-stimulate the T cells when the antigen binding domain is bound to the antigen. Examples of the costimulatory proteins may include CD27,CD28, 4-1BB, OX40, CD30, CD40L, CD40, PD-1, PD-L1, ICOS, LFA-1, CD2, CD7, CD160, LIGHT, BTLA, TIM3, CD244, CD80, LAG3, NKG2C, B7-H3, and a ligand that specifically binds with CD83.

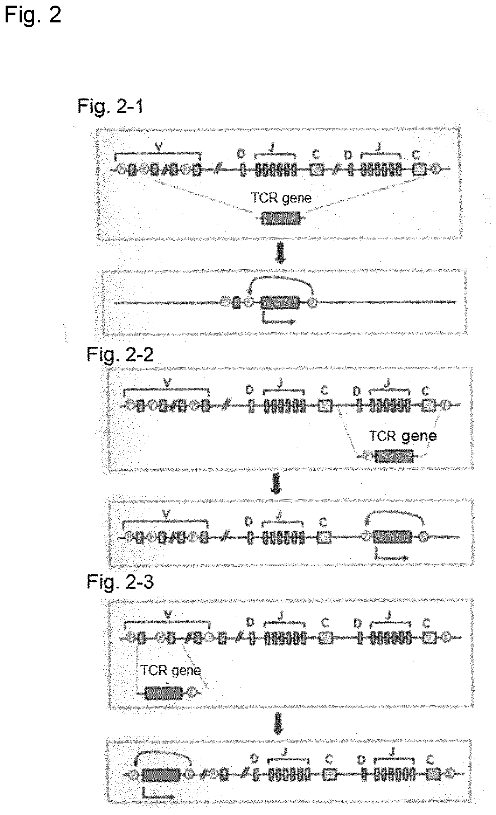

In the present application, as outlined in FIG. 2, there are three methods of introducing an exogenous TCR/CAR gene into a TCR locus of a material cell that is capable of being differentiated into a T cell and has unrearranged TCR locus, and expressing the gene under the TCR expression control system of said material cell:
(1) Introduce a gene containing an exogenous TCR/CAR gene between a C region enhancer and a V region promoter in a TCR locus in the material cell so that the distance between the enhancer and promoter becomes close each other (Fig. 2-1).
(2) Introduce a gene including, in order from upstream to downstream, a V region promoter of a TCR locus and an exogenous TCR/CAR gene into upstream of a C region enhancer of a TCR locus in the material cell so that the V region promoter and the C region enhancer are sufficiently close to each other to exert the TCR expression control system to express the gene sandwiched between them (Figure 2-2).
(3) Introduce a gene including, in order from upstream to downstream, an exogenous TCR/CAR gene and a C region enhancer of a TCR locus into downstream of a V region promoter in a TCR locus in the material cell, so that the V region promoter and the C region enhancer are sufficiently close to each other to exert the TCR expression control system to express the gene sandwiched between them (Figure 2-3).

In the context of "the V region promoter and the C region enhancer are sufficiently close to each other to exert the TCR expression control system to express the gene sandwiched between them", the distance between them is not particularly limited as long as the V region promoter is controlled by the C region enhancer. It is exemplified that the distance between the V region promoter and the C region enhancer after introduction of the exogenous TCR/CAR gene is, for example, about 8 to 50 kbp, about 10 to 40 kbp, about 12 to 32 kbp, or about 14 to 22 kbp.

Cells that have not undergone genetic rearrangement of the TCR loci and are capable of inducing differentiation into T cells are suitably used as material cells for the method of the present application. The following three requirements must be met for "Cells that have not undergone genetic rearrangement of the TCR loci and are capable of inducing differentiation into T cells": (1) cells that have not undergone genetic rearrangement of the TCR loci, (2) cells that are capable of inducing differentiation into T cells, and (3) cells that can withstand the selection process during genetic modification. Such cells include pluripotent stem cells, such as ES cells and iPS cells, and leukocyte stem cells.

Pluripotent stem cells, as used herein and in the claims, are stem cells that are pluripotent, capable of differentiating into many types of cells that exist in living organisms, and that are capable of self-renewal. Pluripotent stem cells include, for example, Embryonic Stem (ES) cells, embryonic stem (ntES) cells derived from cloned embryos obtained by nuclear transfer, sperm stem cells ("GS cells"), embryonic germ cells ("EG cells"), Induced Pluripotent Stem (iPS) cells, and pluripotent cells derived from cultured fibroblasts and bone marrow stem cells ("Muse cells"). ES cells and iPS cells are suitably used. Considering the use of human-derived cells with specific HLAs to produce a cell bank for therapy, it is preferable to use iPS cells.

As for iPS cells, they may be derived from somatic cells of any part of the body. The method to induce iPS cells from somatic cells is well known. iPS cells can be obtained by introducing Yamanaka factors into somatic cells (Takahashi and Yamanaka, Cell 126, 663-673 (2006), Takahashi et al., Cell 131, 861-872(2007) and Grskovic et al., Nat. Rev. Drug Dscov. 10,915-929(2011)). The reprogramming factors used in the induction of iPS cells are not limited to the Yamanaka factors, but any factors or methods known to those skilled in the art may be used.

The introduction of the rearranged TCR/CAR gene into the material cells can be performed in a single operation or proceed in multiple steps. It can also be performed by conventionally known recombination technologies, such as homologous recombination technology, genome editing technology, and technology that uses a combination of recombinases such as Cre recombinase and Flippase recombinase.

If the exogenous TCR/CAR is a heterodimer of TCRα and β, the TCR locus in which the TCR/CAR gene is introduced in the material cell genome may be either TCRα locus or TCRβ locus. It is possible to introduce both rearranged TCRα and TCRβ genes under the TCR expression control system of one gene locus. Alternatively, the TCRα and TCRβ genes may be introduced into the TCRα and TCRβ loci, respectively.

In the present application, the cassette deck/cassette tape method can be used to introduce the TCR/CAR gene. For exchanging tapes, the Recombinase Mediated Cassette Exchange (RMCE) method, which uses a combination of recombinases and their target sequences, such as Cre/lox and Flippase (FLP)/Frt, can be used. In the RMCE method, a cassette exchange reaction between target genes flanked by specific target sequences is induced. The cassette tape section shall be constructed to include an array for cutting and pasting as appropriate. A cassette tape containing the TCR/CAR gene can be introduced into the material cell from the beginning, or a cassette tape containing a drug resistance gene can be introduced first to construct a cassette deck structure, and then a cassette tape including the exogenous TCR/CAR gene can be introduced. That is, the cassette deck can be incorporated under the physiological expression control system of a TCR locus in a material cell, and then various types of TCR/CAR gene can be introduced into one type of material cell by this method.

In one aspect, the present application provides a material cell for introducing an exogenous antigen receptor gene, wherein the material cell comprises in a TCR locus of the genome, in order from upstream to downstream, a V region promoter of a TCR locus, a drug resistance gene or a reporter gene, or a known TCR or CAR gene, a C region enhancer of a TCR.

The material cell may include a target sequence (i) for a recombinase between the V region promoter in the TCR locus and the drug resistance gene or the reporter gene, or the known TCR or CAR gene, and a target sequence (ii) for the recombinase that is differ from the target sequence (i) between the drug resistance gene or the reporter gene, or the exogenous TCR or CAR gene and the C region enhancer in the TCR locus.

An exogenous TCR/CAR gene is introduced into the material cell for introducing an exogenous antigen receptor gene provided in this aspect so as to exchange it with the drug resistance gene, the reporter gene or the known TCR or CAR gene ("cassette exchange"). Cells that have successfully exchanged cassettes can be selected by using the drug resistance gene, the reporter gene, or the known TCR or CAR gene as indicator of negative control. In the case of a drug resistance gene, cells that have not undergone cassette exchange with the exogenous TCR/CAR gene are selected by culturing the cells in the presence of the drug to which the gene is resistant. In the case of a reporter gene, cells in which the gene is expressed can be isolated to remove the cells in which cassette exchange has not occurred. In the case of a known TCR or CAR gene, antibodies or tetramers specific for the gene can be used to remove the cells in which cassette exchange has not occurred.

As for the material cell for introducing an exogenous antigen receptor gene, the cell may have a two-step confirmation system that confirms that the empty cassette has been correctly introduced into the material cell, and further confirms followed by introduction of the foreign TCR/CAR gene that the foreign TCR/CAR gene has been correctly introduced.

As one embodiment of the material cell for introducing an exogenous antigen receptor gene having such a two-step confirmation system, the present application provides a material cell for introducing an antigen receptor gene, comprising in a TCR locus of the material cell genome, in order from upstream to downstream, a V region promoter in a TCR locus, a target sequence (i) for a first recombinase, a promoter that can be expressed in the material cell, a first drug resistance gene linked to be expressed under the promoter that can be expressed in the material cell, a target sequence (ii) for the first recombinase that differs from the target sequence (i), a second drug resistance gene and a target sequence for a second recombinase, and an enhancer of the C region of a TCR locus.

As used herein and in the claims, a "recombinase" is an enzyme that induces site-specific recombination, and a target sequence for a recombinase is a sequence that is recognized by the recombinase and is capable of inducing deletion, incorporation, or inversion between two target sequences. Examples of combinations of recombinase and its target sequences include Cre recombinase and loxP and its derivatives, FLP recombinase and frt, and clonase and attB/attP/attL/attR.

When a first recombinase and a second recombinase are used in the method of the present application, the first recombinase used is an enzyme that can induce recombination specific to a plurality of target sequences, such as Cre recombinase and its target sequences loxP, lox2272, lox loxP, lox2272, lox511, and loxFas. In the presence of Cre recombinase, recombination between identical target sequences is promoted, respectively. By the recombination, for example, the sequence flanked by lox2272 and loxP in the material cell genome can be exchanged with a sequence flanked by lox2272 and loxP on the vector.

The combination of the second recombinase and its target sequence is not particularly limited. Any recombinase that does not have cross-reactivity with the first recombinase, for example, the FLP/frt system may be used.

The "C region enhance of a TCR locus" and the "V region promoter of a TCR locus" may be sequences derived from the material cell, from cells of other individual of the same species of animal as the material cell, or from cells of other species of animal.

The promoter that can be expressed in the material cell is not limited and may be any promoter that can induce expression of the drug resistance gene linked to it in the material cell. Examples include, but are not limited to, cytomegalovirus (CMV) promoter, simian virus 40 (SV40) promoter, and phosphoglycerate kinase (PGK) promoter. The promoter of the mouse phosphoglycerate kinase (PGK) gene (pPGK) is an example.

As a drug resistance gene, a known drug resistance gene that can function as a marker in the material cell can be used. For example, a gene resistant to hygromycin, puromycin, or neomycin may be used. When a first and a second drug resistance genes are used, the combination of the two genes is not limited as long as there is no cross-reactivity between them. The downstream of the drug resistance gene may preferably be linked to a poly A sequence.

The second drug resistance gene is preferably a fusion gene with a drug-sensitive gene downstream thereof. A drug-sensitive gene is a gene that, when expressed, can induce apoptosis of the cells in response to an externally added substance. Such drug-sensitive genes can be selected from known ones as appropriate, and are not particularly limited. For example, thymidine kinase genes of herpes simplex virus and varicella zoster virus may be used. Ganciclovir is an example of a substance that induces apoptosis in the cells incorporating such a gene.

The second drug resistance gene sequence is preferably one in which the initiation codon has been removed to avoid expression of the gene prior to the cassette exchange.

A method for producing a material cell for introducing an antigen receptor gene is described below. There are three possible ways to create a "cassette deck" that includes a so-called "empty cassette" into a TCR locus of the material cell, as shown in Figures 2-1, 2-2, and 2-3. The "empty cassette" sequence is exemplified by a drug resistance gene or a reporter gene, or a known TCR or CAR gene. These genes may be sandwiched between the target sequences (i) and (ii) of a recombinase. Alternatively, an "empty cassette" in the material cell for antigen receptor gene transfer with the two-step confirmation system may include a target sequence (i) for a first recombinase, a promoter sequence that can be expressed in the material cell, a first drug resistance gene linked to be expressed under the promoter sequence that can be expressed in that material cell, a target sequence (ii) for the first recombinase that is differ from the target sequence (i), a second drug resistance gene and a target sequence for a second recombinase are exemplified.

Fig. 2-1) Introduce the "empty cassette" between a C region enhancer and a V region promoter in a TCR locus in the material cell so that the enhancer and the promoter are sufficiently close to each other (Fig. 2-1).

Fig. 2-2) The V region promoter and the "empty cassette" of a TCR locus in order from upstream to downstream are introduced into a TCR locus of the material cell so that the V region promoter is sufficiently close to the C region enhancer of the material cell.

Fig. 2-3) The "empty cassette" and the C region enhancer of a TCR locus in order from upstream to downstream are introduced into a TCR locus in the material cell genome downstream of the V region promoter so that the V region promoter and the C region enhancer on the material cell are sufficiently close.

Hereinafter, as an example, the method of Fig. 2-2, wherein the "empty cassette tape" is a sequence comprising a target sequence (i) for a first recombinase, a promoter sequence which can express in the material cell, a first drug resistance gene linked to be expressed under the promoter sequence which can express in the material cell, a target sequence (ii) for the first recombinase that differs from the target sequence (i), a second drug resistance gene and a sequence comprising a target sequence for a second recombinase, is explained in datail. This embodiment comprises the following steps:
(a) preparing a vector comprising a drug resistance gene cassette, which comprises in order from upstream to downstream, a V region promoter sequence of a TCR locus and a target sequence (i) for a first recombinase, a promoter sequence that can be expressed in the material cell, a first drug resistance gene linked to be expressed under the promoter sequence that can be expressed in the material cell, a target sequence (ii) for the first recombinase that differs from the target sequence (i) and a second drug resistance gene;
(b) knocking in the material cell the sequence comprising the V region promoter sequence on the vector of (a) to the second recombinase target sequence; and
(c) selecting a cell that has successfully knocked in the drug resistance gene cassette, comprising culturing the cells obtained in (b) in the presence of the drug to which the first drug resistance gene is resistant.

As vectors used in the present application, vectors used in genetic recombination may be used as appropriate, for example, vectors such as viruses, plasmids, and artificial chromosomes. Examples of viral vectors include retrovirus vectors, lentivirus vectors, adenovirus vectors, adeno-associated virus vectors, and Sendai virus vectors. The artificial chromosome vectors include, for example, Human Artificial Chromosomes (HAC), Yeast Artificial Chromosomes (YAC), and bacterial artificial chromosomes (BAC, PAC). As plasmid vectors, plasmid vectors for mammalian cells may be used. Commercially available vectors may be selected and used according to the purpose.

The TCR locus on the material cell genome at which the drug resistance gene cassette is knocked in may be the TCRα or TCRβ locus when producing αβ T cells. The TCRα and β locus that is not used for gene transfer may preferably be deleted. Deletion of a specific gene locus can be performed using known methods as appropriate, and can be performed using known genome editing techniques, such as CRISPR/Cas9 and Talen.

In this embodiment, the following step (a) may be conducted first:
Constructing a vector comprising a drug resistance gene cassette which comprises in order from upstream to downstream, a V region promoter of a TCR locus and a target sequence (i) for a first recombinase, a promoter that can be expressed in the material cell, a first drug resistance gene linked to be expressed under the promoter that can be expressed in the material cell, a target sequence (ii) for the first recombinase and a second drug resistance gene and a target sequence for the second recombinase, followed by constructing "drug resistance gene cassette knock-in targeting vector" which comprises a V region promoter upstream of the drug resistance gene cassette.

A targeting vector comprising the V region promoter of a TCR locus upstream of the drug resistance gene cassette is then constructed.

In the V region of a TCR locus, there is one promoter for each V gene. The V region promoter of the TCR locus used is not limited and may be selected as appropriate. For example, in the case of using the TCRβ locus, the Vβ20-1 promoter is exemplified. The promoter can be obtained by amplifying the sequence by PCR with primers designed to obtain a DNA fragment of the promoter upstream from just before the translation start point in the first exon of the V gene.

In constructing the targeting vector, the site in the material cell genome TCR locus where the exogenous TCR/CAR gene will be introduced is determined first. The site for introduction of the exogenous gene can be any site where the C region enhancer of the material cell can activate the V region promoter when the sequence containing in order from upstream to downstream, the V region promoter and the exogenous TCR/CAR gene is introduced.

Specifically, when introducing an exogenous TCR/CAR gene into the TCRβ locus of a material cell, it is preferable to introduce the gene in a region where the TCRβ locus has not been rearranged and is close to the enhancer, such as a site upstream of Dβ2 and downstream of Cβ1. When introducing an exogenous TCR/CAR gene into the TCRα locus of a material cell, it is preferable to introduce the gene in a region where the TCRα locus has not been rearranged and is close to the enhancer. Introducing the TCR/CAR gene at a site upstream of the most upstream Jα gene and downstream of the most downstream Vα gene is an example.

Once the site of introduction in a TCR locus of the material cell genome is determined, sequences homologous to the sequences upstream and downstream of the introduction site are introduced as the 5' arm and 3' arm, respectively, to allow homologous recombination. In this context, a "homologous sequence" is sufficient if the sequences of the 5' arm and the 3' arm are homologous to the extent that homologous recombination occurs, respectively.

For example, in the case of introducing an exogenous TCR/CAR gene into a non-rearranged TCRβ locus in the genome of a material cell, a DNA fragment from about 110bp upstream of the Dβ2 gene to about 1.6kbp further upstream is used as the 5' arm sequence, and a DNA fragment from about 50bp upstream of the Dβ2 gene to about 1.6kbp downstream is used as the 3' arm sequence. DNA fragments of each 5' and 3' arm can be obtained by PCR amplification of genomic DNA of the material cell as the template using primers that can specifically amplify each sequence.

In other words, drug resistance gene knock-in targeting vector may comprise, in order from upstream to downstream, a sequence homologous to the 5' side of the introduction site in a TCR locus of the material cell genome (5' arm), a V region promoter in a TCR locus, a target sequence (i) for a first recombinase, a promoter that can be expressed in the material cell, a first drug resistance gene linked to be expressed under the promoter that can be expressed in the material cell, a target sequence (ii) for the first recombinase that differs from the target sequence (i), a second drug resistance gene and a target sequence for a second recombinase, and a sequence homologous to the 3' side of the introduction site (3' arm) of the material cell is exemplified. When each sequence is amplified by PCR, the primers are designed so that the resulting PCR products include DNA sequences required for introducing the same into the drug resistance vector. The obtained PCR product can be used to construct a vector using a known method, such as the Gibson assembly method, or a commercially available kit.

The drug resistance gene cassette knock-in targeting vector may further comprise a promoter that can be expressed in the material cell and a marker gene downstream of the 3' arm. An example of such a promoter-marker combination is the combination of the MC1 promoter and diphtheria toxin (DTA).

### Step (b)

The drug resistance gene cassette knock-in targeting vector is knocked into a TCR locus of the material cell by homologous recombination. Knocking-in the vector can be performed by a known method, for example, by electroporation.

In order to increase the knock-in efficiency by homologous recombination, it is preferable to introduce two single-strand breaks (nicks) near the knock-in site, e.g., the start site (upstream) of the 3' arm, prior to knocking-in the drug resistance gene cassette knock-in targeting vector. The introduction of the nick can be performed by a known method, and the CRISPR/Cas9n system is an example.

### Step (C) :

Material cells that have successfully undergone homologous recombination express the drug resistance gene 1 by the action of the introduced promoter. Therefore, material cells that have successfully undergone homologous recombination can be selected in the presence of the drug to which the drug resistance gene 1 is resistant. In addition, when a marker gene is introduced downstream of the 3' arm of the targeting vector, the material cells introduced with the "outer part of the 5' and 3' arm sequences" that does not contain the drug resistance gene cassette express the marker, for example cytotoxin, and the cells are not viable. Thus, by selecting viable cells, cells into which the drug resistance cassette is successfully knocked-in can be selected. The selected material cells may be further confirmed by PCR to select only the cells in which the V region promoter and the drug resistance gene cassette are knocked in.

A clone obtained from the material cells in which the V region promoter and the drug resistance gene cassette have been knocked-in can be used in the present application as a "material cell for TCR/CAR gene knock-in, comprising in a TCR locus of the material cell genome, in order from upstream to downstream, a V region promoter in a TCR locus, a target sequence (i) for a first recombinase, a promoter that can be expressed in the material cell, a first drug resistance gene linked to be expressed under the promoter that can be expressed in the material cell, a target sequence (ii) for the first recombinase that differs from the target sequence (i), a second drug resistance gene and a target sequence for a second recombinase, and an enhancer of the C region of a TCR locus".

Although the above description is based mainly on the manner of Fig. 2-2, a person skilled in the art will understand that the other two embodiments can be implemented in the same manner. That is, if the V region promoter in the material cell is used, or if both the V region promoter and the C region enhancer in the material cell are used, the construct of the drug resistance gene cassette knock-in targeting vector can be prepared as appropriate for the configuration.

A specific example of the embodiment of Fig. 2-1 is described in Example 3. If both the V region promoter and the C region enhancer in the material cell are used, about 180 kbp genomic DNA can be excluded by introducing two single-strand breaks (nicks), one slightly downstream of the V region promoter and the other slightly upstream of the C region enhancer, e.g., in the case of the TCRβ locus, in a region 30 bp to 80 bp downstream of the translation start site of TCRVβ20-1 and within exon 1 of the TCRCβ2 gene, thereby approximately 180kbp between the two nicks is excluded. Subsequently, the drug resistance gene cassette can be knocked in between the V region promoter and C region enhancer that are close each other to create a material cell for TCR/CAR gene knock-in, or an exogenous TCR/CAR gene can be introduced directly.

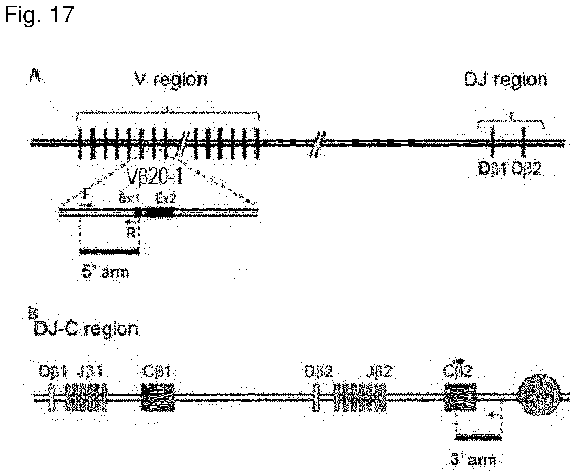

Figs. 17-19 are schematic diagrams of the procedure for obtaining material cells wherein both the V region promoter and the C region enhancer in a TCR locus in the material cell are used, wherein the material cell for TCR/CAR gene knock-in comprising in a TCR locus of the material cell genome, in order from upstream to downstream, a V region promoter in a TCR locus, a target sequence (i) for a first recombinase, a promoter that can be expressed in the material cell, a first drug resistance gene linked to be expressed under the promoter that can be expressed in the material cell, a target sequence (ii) for the first recombinase that differs from the target sequence (i), a second drug resistance gene and a target sequence for a second recombinase, and an enhancer of the C region of a TCR locus can be prepared.

To introduce an exogenous TCR/CAR gene into the material cells for TCR/CAR transduction, the following procedures are illustrated:

(A) preparing a TCR or CAR gene cassette exchange vector comprising, in order from upstream to downstream, a target sequence (i) for a first recombinase, an exogenous TCR or CAR gene, a target sequence for a second recombinase, a promoter that can be expressed in the material cell, and a target sequence (ii) for the first recombinase;
(B) introducing the TCR or CAR gene cassette exchange vector into the material cell for TCR/CAR gene transduction, and simultaneously applying the first recombinase to exchange the portion of the drug resistance gene cassette introduced into the material cell genome that is flanked by the target sequences (i) and (ii) of the first recombinase with the sequence flanked by the target sequences (i) and (ii) of the first recombinase in the TCR or CAR gene cassette exchange vector; and
(C) selecting cells that have successfully exchanged the cassette, comprising culturing the cells in the presence of the drug to which the second drug resistance gene is resistant; and
(D) causing the second recombinase to act on the cell selected in (C) to remove the second drug resistance gene flanked by the target sequence for the second recombinase.

### Step (A)

To introduce the rearranged TCR or CAR gene, a TCR cassette exchange vector containing, in order from upstream to downstream, a target sequence (i) for a first recombinase, the exogenous TCR or CAR gene, a target sequence for a second recombinase, a promoter that can be expressed in the material cell, and a target sequence (ii) for the first recombinase is prepared first.

The following is an example of a TCR cassette exchange vector for introducing a gene of a heterodimer of TCRα and TCRβ.

In the case of expressing a heterodimer of TCRα and TCRβ as one embodiment of the present application, it is exemplified that a TCR cassette exchange vector is created using a sequence in which the rearranged TCRα gene and TCRβ gene are connected by a self-cleaving 2A peptide. By placing the self-cleaving 2A peptide between the α and β chains, it becomes possible to express both genes under the control of a single gene expression system.

2A peptides such as p2A, T2A, E2A, and F2A can be used, and p2A peptide, which is said to have good cleavage efficiency, is suitable. Either the TCRα gene or the TCRβ gene may be introduced upstream, and the poly A sequence is suitably linked to the TCR gene introduced downstream.

An intron is preferably included upstream of the TCR gene. The intron may include a splice donor sequence and a splice acceptor sequence in addition to the sequence to be removed by splicing. The intron of the human polypeptide chain elongation factor alpha (EF1α) gene or the intron of the chicken beta-actin (CAG) gene promoter are exemplified.

If the second drug resistance gene does not contain an initiation codon, the initiation codon is introduced in the TCR cassette exchange vector immediately after the promoter that can be expressed in the material cell and immediately before the target sequence (ii) for the first recombinase.

### Step (B)

Introduce the TCR cassette exchange vector into the material cells for TCR/CAR transduction and simultaneously apply the first recombinase to the material cells. To apply the first recombinase, for example, the TCR cassette exchange vector is introduced together with the first recombinase expression vector. Expression of the first recombinase expression vector facilitates recombination between the target sequence (i) on the drug resistance gene cassette and the target sequence (i) on the TCR cassette exchange vector, and between the target sequence (ii) on the drug resistance gene cassette and the target sequence (ii) on the TCR cassette exchange vector, respectively. As a result, the part flanked by the target sequences (i) and (ii) of the first recombinase on the drug resistance gene cassette is exchanged with the part flanked by the same sequences on the TCR cassette exchange vector.

### Step (C)

In the material cells where cassette exchange occurs, the second drug resistance gene is expressed. Thus, by culturing the material cells after cassette exchange in the presence of the drug to which the second drug resistance gene is resistant, cells that have successfully exchanged the cassette can be selected.

### Step (D)

The second recombinase is applied to the selected cells. To apply the second recombinase, for example, a second recombinase expression vector can be introduced into said selected cells.

Expression of the second recombinase results in deletion of the part flanked by the target sequence for the second recombinase. If the introduced second drug resistance gene is fused with a drug-sensitive gene, culturing the material cells in the presence of a factor that triggers activation of the drug-sensitive gene, e.g. a cell death-inducing gene, removes cells that fail to delete the part flanked by the target sequence for the second recombinase. The PCR method may be used to confirm that the TCRα and TCRβ genes have been definitely introduced into the obtained cells. The cells obtained by the above method express both TCRα and TCRβ in the TCRβ locus of the material cell.

### Differentiation of the material cells transfected with the exogenous TCR/CAR gene into T cells

The TCR genes at the TCR loci in cells other than T cells will not be expressed because the TCR gene rearrangement will not occur. Therefore, rearranged TCR genes or CAR genes are used for gene transfer. When cells other than T cells, such as pluripotent stem cells, are used as material cells, they must be differentiated into T cells in order to express the rearranged TCR or CAR gene, and the differentiated T cells can be used for the cell therapy. Methods for inducing differentiation of pluripotent stem cells into T cells are exemplified in Timmermans et al, Journal of Immunology, 2009, 182: 6879-6888, Nishimura T et al, 2013, Cell Stem Cell 114-126, WO 2013176197 A1, and WO 2011096482 A1. Upon the differentiation, the Rag 1 and Rag 2 genes may be deleted to suppress the rearrangement of the TCR. For the Rag 1 and Rag 2 genes, it is only necessary to delete one or the other.

A T cell is a cell that expresses CD3 and at least one of CD4 and CD8. Depending on the therapeutic purpose, the cells may be differentiated into either CD8-expressing killer T cells or CD4-expressing helper T cells.

The cells obtained by the method of the present application can be used for the treatment of immune-mediated diseases such as cancer, infectious diseases, autoimmune diseases, and allergies that express antigens to which the introduced TCR or CAR specifically binds. In the method of the present application, the resulting T cells are suspended in an appropriate medium, such as physiological saline or PBS, and used to treat patients whose HLA matches the donor from which the material cells are derived to a certain degree. The HLA types of the donor and the patient may be perfectly matched, or at least one of the HLA haplotypes is matched if the donor has homozygous HLA haplotype. Of course, pluripotent stem cells derived from the patient's own somatic cells may be used as material cells. Administration of the cells to the patient should be done intravenously.

For example, iPS cells may be those having an HLA haplotype that matches at least one of the HLA haplotypes of the subject to be treated and selected from an iPS cell bank in which iPS cells established from cells of donors with a homozygous HLA haplotype are stored in connection with information regarding HLA of each donor.

The number of the cells to be administered is not particularly limited and may be determined as appropriate according to the patient's age, sex, height, weight, target disease, symptoms, etc. The optimal number of the cells may be determined by clinical trials.

The method of the present application can be used to induce antigen-specific T cells or antigen-specific CAR-T cells. The method of this application can be applied to immune cell therapy for various diseases such as cancer, infectious diseases, autoimmune diseases, and allergies.

In another aspect, the present application provides a method for producing cells for cell therapy in which exogenous TCRα and TCRβ genes are introduced between a V region promoter and a C region enhancer in a TCR locus of a T cell having genetically rearranged TCRs (material cell). The exogenous TCRα and TCRβ genes are introduced under the expression control system of either TCRα or TCRβ expressed on the T cells, which are the material cells, so that both introduced TCR genes are expressed. TCR expression system that is not used to introduce the exogenous TCRα and TCRβ genes may be defected.

In this aspect, the material cells for introducing an exogenous antigen receptor gene described above are differentiated into T progenitor cells or T cells, and then the exogenous TCR or CAR gene is introduced between the V region promoter and the C region enhancer in the TCR locus of the induced T progenitor cells or T cells by genome editing or Recombinase-mediated Cassette Exchange (RMCE).

For example, if the material cells for the introduction of an exogenous antigen receptor gene have a known TCR or CAR gene, the known TCR or CAR gene in the induced T progenitor cells or T cells may be replaced with an exogenous TCR or CAR gene by genome editing or Recombinase-mediated Cassette Exchange (RMCE), and then, cells whose known TCR or CAR gene has not been successfully replaced with the exogenous TCR or CAR gene are excluded using an antibody or tetramer specific for the known TCR or CAR gene.

As described in the above step-by-step instructions, the present application also provides a method for producing cells comprising an exogenous TCR or CAR gene, which includes the steps of:
(1) preparing a vector comprising a drug resistance gene cassette comprising in order from upstream to downstream, a V region promoter in a TCR locus, a target sequence (i) for a first recombinase, a promoter that can be expressed in a material cell, a first drug resistance gene linked to be expressed under the promoter that can be expressed in the material cell, a target sequence (ii) for the first recombinase that differs from the target sequence (i), a second drug resistance gene, and a target sequence for the second recombinase,
(2) knocking-in the sequence of (1) into a TCR locus on the material cell genome;
(3) culturing the cells obtained in (2) in the presence of a drug to which the first drug resistance gene is resistant to select the cells to which the drug resistance gene cassette has successfully been knocked-in;
(4) preparing a TCR or CAR gene cassette exchange vector comprising, in order from upstream to downstream, the target sequence (i) for the first recombinase, an exogenous TCR or CAR gene, the target sequence for the second recombinase, a promoter that can be expressed in the material cell, and the target sequence (ii) for the first recombinase;
(5) introducing the TCR or CAR gene cassette exchange vector into the material cells selected in step (3) in which the drug resistance cassette has been knocked-in, and simultaneously applying the first recombinase to the material cells so that the sequence in the drug resistance gene cassette is replaced with the sequence flanked by the target sequences (i) and (ii) of the first recombinase in the TCR or CAR gene cassette exchange vector;
(6) applying the drug to which the second drug resistance gene is resistant to the cells to select the cells that has successfully exchanged the cassette;
(7) applying the second recombinase on the cell selected in (6) to remove the second drug resistance gene part flanked by the target sequence for the second recombinase.

The present application also provides, as an example of a method for efficiently creating T cells transfected with a desired TCR or CAR gene, a method comprising the steps of:
(1) preparing a vector comprising a drug resistance gene cassette comprising in order from upstream to downstream, a V region promoter in a TCR locus, a target sequence (i) for a first recombinase, a promoter that can be expressed in a material cell, a first drug resistance gene linked to be expressed under the promoter that can be expressed in the material cell, a target sequence (ii) for the first recombinase that differs from the target sequence (i), a second drug resistance gene, and a target sequence for the second recombinase,
(2) knocking-in the sequence of (1) into a TCR locus on the material cell genome;
(3) culturing the cells obtained in (2) in the presence of a drug to which the first drug resistance gene is resistant to select the cells to which the drug resistance gene cassette has successfully been knocked-in;
(4) preparing a known TCR or CAR gene cassette exchange vector comprising, in order from upstream to downstream, the target sequence (i) for the first recombinase, a knownn exogenous TCR or CAR gene, the target sequence for the second recombinase, a promoter that can be expressed in the material cell, and the target sequence (ii) for the first recombinase;
(5) introducing the known TCR or CAR gene cassette exchange vector into the material cells selected in step (3) in which the drug resistance cassette has been knocked-in, and simultaneously applying the first recombinase to the material cells so that the sequence in the drug resistance gene cassette is replaced with the sequence flanked by the target sequences (i) and (ii) of the first recombinase in the known TCR or CAR gene cassette exchange vector;
(6) applying the drug to which the second drug resistance gene is resistant to the cells to select the cells that has successfully exchanged the cassette;
(7) differentiating the selected sells into T cells;
(8) preparing a desired TCR or CAR gene cassette exchange vector comprising, from upstream to downstream, the target sequence (i) for the first recombinase, the desired TCR or CAR gene, target sequence for the second recombinase, a promoter that can be expressed in the material cell, and the target sequence (ii) for the first recombinase;
(9) introducing the desired TCR or CAR gene cassette exchange vector into the T cells obtained in step (7) and simultaneously applying the first recombinase to exchange the desired TCR or CAR gene flanked by the target sequences (i) and (ii) in the desired TCR or CAR vector with the known TCR or CAR gene in the T cells;
(10) applying the second recombinase on the cell selected in (9) to remove the second drug resistance gene part flanked by the target sequence for the second recombinase.

### EXAMPLE 1

The present application will be described in more detail referring to the examples below. In the examples of the present application, a T cell line, Jurkat cells were used. Jurkat cells have both a TCR locus that has not been completely rearranged (until D-J recombination) and a rearranged TCR locus (VDJ recombination). In this example, Jurkat cells with the rearranged TCR locus disrupted were used.

### Expression of exogenous TCR by the cassette exchange method in the T cell receptor (TCR) gene-disrupted Jurkat cells

### 1) Reagents, antibodies, and etc:

KOD-Plus-Neo (Toyobo, KOD-401), Amaxa (registered trademark) Cell Line Nucleofector (registered trademark) Kit V (Lonza, VACA-1003), Gibson assembly master mix (New England Biolabs (NEB, E2611S), Gateway (registered trademark) LR ClonaseTMII enzyme mix (Thermo Fisher Scientific, 11791-020), Hygromycin B Gold (InvivoGen,ant-hg-1), Puromycin dihydrochloride (Wako,160-23151), ganciclovir (Wako,078-04481), PE/Cy7 anti-human TCRα/β (BioLegend,306719), and APC Mouse Anti-Human CD3 (BD Pharmingen,557597) were used.

In addition, pBRBlII-AscI_FRTPGKpacΔtkpA_AscI was used as a vector.

Jurkat cells are a T cell line derived from human leukemia cells. A strain in which a TCR gene was disrupted by irradiation (J.RT3-T3.5) was used (J. Exp. Med. 160. 1284-1299. 1984).

### 2) Construction of a drug resistance gene cassette vector

As a drug resistance gene cassette vector as shown in Fig. 3G, the lox2272 sequence (5'-ATAACTTCGTATAAAGTATCCTATACGAAGTTAT-3' (SEQ ID NO: 1)), which is the target sequence for Cre recombinase, mouse phosphoglycerate kinase (PGK) gene promoter (pPGK), hygromycin resistance gene (Hygror) downstream of the PGK gene, poly adenylation sequence (pA) of the PGK gene, and loxP sequence (5 '-ATAACTTCGTATAGCATACATTATACGAAGTTAT-3' (SEQ ID NO: 2)) which is the target sequence for Cre recombinase differ from lox2272, a fused gene between the puromycin resistance gene and the kinase domain of herpesvirus thymidine kinase (ΔTK) (PurorΔ TK), the pA sequence, and the frt sequence, which is the target sequence for the yeast recombinase Flippase (FLP), were linked to construct the plasmid.

First, primer 1 consisting of "sequence identical to the vector (about 15bp)" - "lox2272 " - "PGK promoter 5' side sequence" as shown in Figure 3A was designed. Primer 2 was designed as "Identical sequence with vector" - "Sequence on 3' side of the PGK promoter" and amplified by PCR using pBRBlII-AscI_FRTPGKpacAtkpA_AscI vector as template (Figure 3A). The pBRMC1DTApA vector was then cleaved with the restriction enzyme XbaI as shown in Fig. 3B. Primer 1 has the same DNA sequence as the 5' side sequence-1 at the site of the vector cleaved with restriction enzyme XbaI, and primer 2 has the same DNA sequence as the 3' side sequence-2 of about 15bp of the same cleaved site (Fig. 3A,B). The PCR products and restriction enzyme XbaI-cleaved vectors shown in Fig. 3A and B, respectively, were linked by Gibson assembly master mix (E2611S, New England Biolabs (NEB)) through approximately 15 bp of identical sequence to obtain the PGK vector with the structure shown in Fig. 3C. Gibson assembly was carried out according to the Gibson assembly master mix manual.

Next, as shown in Fig. 3E and F, primer 3 (Fig. 3E), which includes sequence-3 identical to a part of the PGK vector sequence (Fig. 3D) and a part of the 5' side of the hygroimycin resistance gene, and primer 6 (Fig. 3F), which includes sequence-4 identical to a part of the PGK vector and a part of the frt sequence were designed. Primer 4 and primer 5 (Fig. 3E,F) were designed to have sequences that are homologous to each other, with primer 4 having the loxP sequence and part of the pA sequence, and primer-2 having part of the sequence of the puromycin resistance gene (without the start codon). Amplification was performed by PCR using PB-flox (CAG-mCherry-IH;TRE3G-miR-155-LacZa) vector as the template and using primer 3 and primer 4 (Fig. 3E).

Similarly, primer 5 and primer 6 were used to PCR using the pBRBlII-AscI_FRTPGKpacΔtkpA_AscI vector as the template (Fig. 3F). The PGK vector in Fig. 3C was then cleaved with restriction enzyme XbaI (Fig. 3D), and the PCR products generated in Figs. 3E and F were linked by the Gibson assembly method to obtain the drug resistance gene cassette vector (Fig. 3G).

The sequences of the primers are as follows.
Primer 1: 
5′-GCGGTGGCGGCCGCTATAACTTCGTATAAAGTATCCTATACGAAGTTATTACCGGGT
AGGGGAGGCGCTT-3′ (SEQ ID NO: 3),
primer 2:5'-GGGGATCCACTAGTTCTAGACGAAAGGCCCGGAGATGAGGA-3' (SEQ ID NO: 4),
primer 3: 5'-CTCCGGGCCTTTCGTGCCACCATGAAAAAGCCTGAACTCACC-3' (SEQ ID NO: 5),
primer 4: 5'- 
ATAACTTCGTATAATGTATGCTATACGAAGTTATCAACTATGAAACATTA
TCATA-3′ (SEQ ID NO: 6)
primer 5: 5'-ATTATACGAAGTTATCCACCGAGTACAAGCCCACGGTG-3' (SEQ ID NO: 7),
primer 6: 5'-GGGGATCCACTAGTTGAAGTTCCTATACTTTCTAGA-3' (SEQ ID NO: 8)

PCR was performed using KOD-Plus-Neo (KOD-401, TOYOBO) at 1) 94°C for 2 min, 2) 98°C for 10 sec, 3) 50 to 60°C for 30 sec, and 4) 68°C for 2 min, with 30 to 35 cycles of 2) to 4).

The start codon in the puromycin resistance gene was removed to avoid expression prior to be subjected to the cassette exchange (primer 5). Downstream of Frt, a MC1 promoter and a diphtheria toxin gene (DTA) were incorporated (Fig. 3G).

### 3) Construction of a drug resistance gene cassette knock-in targeting vector (1)

Acquisition of 5' arm and 3' arm, and promoter DNA fragments A DNA fragment from a position approximately 110bp upstream of the Dβ2 gene in the human T cell receptor β (TCRβ) (TCRDβ2) to a site approximately 1.6 kbp further upstream was designated as 5' arm, and a DNA fragment from a site approximately 50 bp upstream of TCRDβ2 to a site approximately 1.6 kbp downstream was designated as 3 'arm (Fig. 4A). Each DNA fragment was amplified by PCR using the genomic DNA of the Jurkat cell as the template with the primers shown below. As an endogenous V gene promoter, the promoter of the TCRβV20-1 gene (Vβ20-1 promoter) was used in this example. For the Vβ20-1 promoter, a DNA fragment from just before the translation start site in the first exon upstream to approximately 1.6 kbp was amplified. (Fig. 4B).

Each primer used for PCR had a DNA sequence added so that the resulting PCR product can be introduced into the drug resistance cassette vector. The vector was constructed using the Gibson assembly method described above via the added sequences. The primer sequences used are as follows.

Primer for 5' arm (about 1.6 kbp) acquisition
Primer 5'-1: 5'-CTCCACCGCGGTGGCGGCCGCCTTCAAAAGCTCCTTCTGTTGT-3' (SEQ ID NO: 9)
Primer 3'-1: 5'-ATGATGCGGCCGCTAGCCTTGGAAAAGACAAAGGCAGT-3' (SEQ ID NO: 10)

Primer for 3' arm (about 1.6kbp) acquisition
Primer 5'-2: 5'-AGGAATTCGATATCATTTAAATGAGGGGGACTAGCAGGGAGGA-3' (SEQ ID NO: 11)
Primer 3'-2: 5'-TCGACGGTATCGATATTTAAATCCCCGAAAGTCAGGACGTTG-3'(SEQ ID NO: 12)

Primer for Vβ20-1 promoter (about 1.6 kbp) acquisition
F: 5'-GCTAGCGGCCGCATCATGAGACCATCTGTACCTG-3' (SEQ ID NO: 13)
R: 5'-ATACGAAGTTATAGCTAGTCTTCCGTGATGGCCTCACACCA-3'(SEQ ID NO: 14)

PCR was performed with KOD-Plus-Neo using genomic DNA of the Jurkat cell as the template by 1) 95°C, 3 min → 2) 98°C, 10 sec → 3) 50 to 60°C, 30 sec → 4) 68°C, 1 to 3 min, and 30 to 35 cycles of 2) to 4) were performed.

### 4) Construction of drug resistance gene cassette knock-in targeting vector II

Construction of drug resistance gene cassette knock-in (KI) targeting vector for knocking into the Dβ2 region of the TCRβ locus

The vector was cleaved at a site outside (downstream) the frt site of the drug resistance gene cassette with the restriction enzyme HindIII (Fig. 5, A), and the 3' arm DNA fragment was placed therein using the Gibson assembly method according to the manual. (Fig. 5 B). Next, outside (upstream) of lox2272 in the drug resistance gene cassette in the vector was cleaved with the restriction enzyme NotI (Fig. 5,C), and the 5' arm DNA fragment and the Vβ20-1 promoter DNA fragment were introduced simultaneously using the Gibson assembly method (Fig. 5D).

The resulting drug resistance gene cassette knock-in targeting vector (KI) (Drug Resistance Gene KI Targeting Vector, 7) was used in the following procedures.

### 5) Knocking-in of the drug resistance gene cassette into the Pβ2 region of the TCRβ locus in the Jurkat cell by homologous recombination.

Generation of the drug resistance gene knocked-in (KI)-Jurkat cells

The culture medium shown below was used for cell culture in all examples. In the case of selection by an agent, each agent was added to the composition shown below and cultured.

**[Table 1]**

| Cell culture medium | (final concentration) |
|---|---|
| RPMI1640 | 500 mL |
| FCS | 50 mL (9%) |
| * penicillin/streptomycin/L-glutamine solution | 5.55 mL(1%) |
| 2-mercaptoethanol | 2 µL (50 µM) |
| Total | 555 mL |

| | |
|---|---|
| *The composition of the penicillin/streptomycin/L-glutamine solution is 10,000 U/mL of penicillin, 10,000 µg/mL of streptomycin, and 29.2 mg/mL of L-glutamine, so the final concentrations are 100 U/mL, 100 µg/mL, and 292 µg/mL, respectively. | |

The vector was introduced into the cells by electroporation. Electroporation was performed according to the manual of the Amaxa®CellLineNucleofector®Kit V. The cells and the vector were suspended in the reagents provided in the kit, the suspension was transferred to the cuvette provided in the kit, set in the AmaxaNucleofectorII (Lonza), and the vector was introduced into the cells using the built-in program X001.

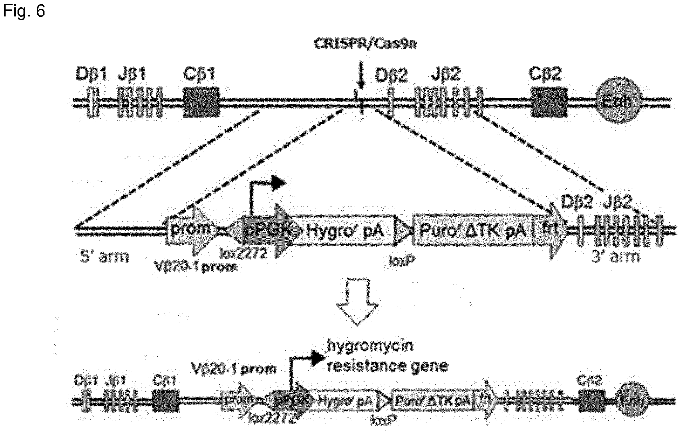

The Vβ20-1 promoter and drug resistance gene cassette were introduced into the approximately 50bp upstream of the Dβ2 gene in the TCRβ locus of the T cell line, Jurkat cell (Fig. 6, upper panel), using the drug resistance gene KI targeting vector (Fig. 6, middle panel). The schematic diagram of this example where the vector was introduced is shown in the lower panel of Fig. 6.

To increase the efficiency of knocking-in by homologous recombination, two single-strand breaks (nicks) were introduced at approximately 50bp upstream of the Dβ2 gene by the CRISPR/Cas9n system. The KI-targeting vector was introduced together with two CRISPR/Cas9n vectors (see material below) to J.RT3-T3.5 Jurkat cells, a variant of the Jurkat cell line with impaired expression of the endogenous TCRβ gene (hereafter referred to as Jurkat β mutant) (J. Exp. Med. 160. 1284-1299. 1984).

In the cells into the genomic DNA of which the drug resistance gene cassette was incorporated by homologous recombination, the hygromycin resistance gene (the first drug resistance gene) was expressed by the PGK promoter. The puromycin resistance gene, the second drug resistance gene, was not expressed here. (Fig. 6, lower panel). Clones that were incorporated with the drug resistance gene cassette into their genome were selected using 250 µg/mL hygromycin/culture medium (positive selection).

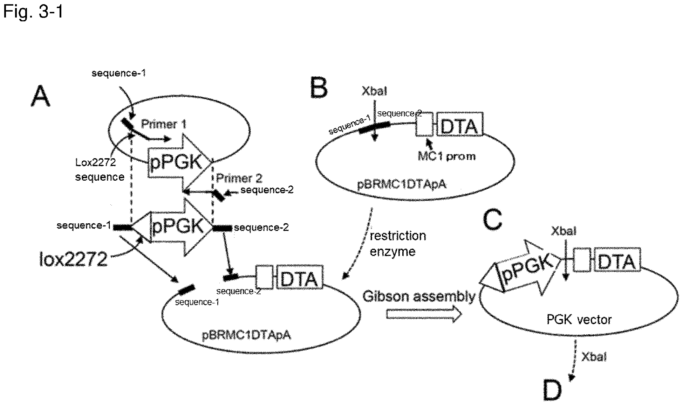

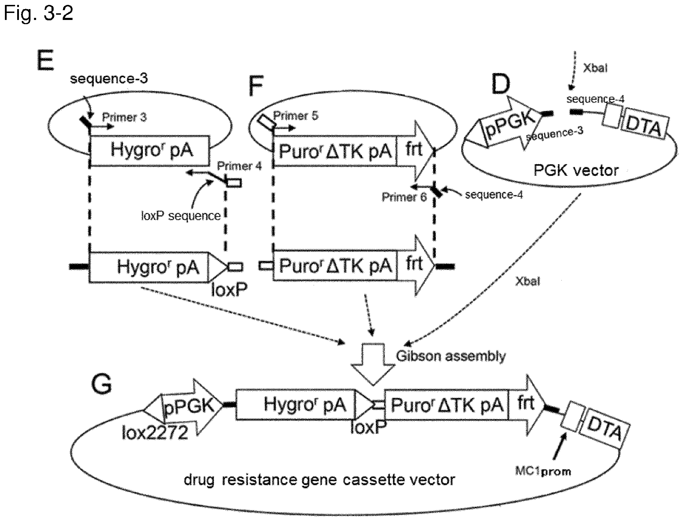

On the other hand, cells in which the outer part of the 5'-arm and 3'-arm sequences of the drug resistance gene KI targeting vector was incorporated into their genomic DNA by random integration could not survive because diphtheria toxin (DTA) (Fig. 3) was produced in the cells and therefore were removed (negative selection). Then, from the cells (clones) so selected, clones that were incorporated with the part from the Vβ20-1 promoter to frt of the drug resistance gene cassette into the TCRDβ2 locus were identified by PCR. Thus, the clone with the Vβ20-1 promoter and drug resistance gene cassette in the Dβ2 region was selected (drug resistance gene KI-Jurkat cells).

### Materials

**[Table 2]**

| cells | Jurkat β mutant | 1x10⁶ cells |
|---|---|---|
| | drug resistance gene KI targeting vector | 1.2 µg |
| vectors | CRISPR/Cas9n vector 1 | 0.6 µg |
| | CRISPR/Cas9n vector 2 | 0.7 µg |
| agent | Amaxa®CellLineNucleofector®KitV | 100 µL |

CRISPR/Cas9n vectors were prepared by using the following oligonucleotides:
A: 5'-CACCGAGGTTAGTCTGACTGTGTG-3'(SEQ ID NO: 15),
B: 5'-AAACCACACAGTCAGACTAACCTC-3' (SEQ ID NO: 16)
C: 5'-CACCCTGCCGCTGCCCAGTGGTTG-3' (SEQ ID NO: 17)
D: 5'-AAACCAACCACTGGGCAGCGGCAG-3' (SEQ ID NO: 18)
Oligonucleotides A and B (vector 1), and oligonucleotides C and D were annealed, respectively, and then, introduced into the plasmid pX460 cleaved with the restriction enzyme Bbs1.

### 6) Disruption of the TCRα gene in the drug resistance gene KI-Jurkat cells (generation of TCRα-KO drug resistance gene KI-Jurkat cells)

TCRs specifically recognize specific antigen/HLA complexes by means of the unique combination of alpha and beta chains in the individual T cells. In this example, the endogenous TCR α-chain gene was disrupted by the CRISPR/Cas9 system in order to maintain a strict combination of α- and β-chains of the TCR to be introduced into the cells later.

### Materials

**[Table 3]**

| cells | drug resistance gene KI-Jurkat cell | 1x10⁶ cells |
|---|---|---|
| vector | CRISPR/Cas9 vector for TCRα_KO | 2.5 µg |
| agent | Amaxa®CellLineNucleofector®KitV | 100 µL |

CRISPR/Cas9 vectors were prepared by using the following oligonucleotides
E: 5'-CACCGAGAATCAAAATCGGTGAAT-3'(SEQ ID NO: 19)
F: 5'-AAACATTCACCGATTTTGATTCTC-3'(SEQ ID NO: 20)
Oligonucleotides E and F were annealed and then, introduced into the plasmid pX330 cleaved with the restriction enzyme Bbs1.

The vector was introduced into the cells as in step 5). Then, vector-transfected cells were seeded on 96-well plates, on which B6 mouse thymocytes were seeded at a density of 1x10⁵ cells per well, at a density of approximately 0.5 cell per well. After 3 to 4 weeks, clones were isolated, genomic DNA was extracted, and the TCRα gene was analyzed by amplifying the DNA region containing the target site of CRISPR/Cas9 by PCR and deciphering its sequence. As a result, clones that showed disruption of the TCRα gene at both alleles (mutations that caused frameshifts) were identified.
The PCR primers used are as below. Analysis of the sequence of the PCR product was performed with the forward primer. Forward primer: 5'-CCTTGTCCATCACTGGCATC-3' (SEQ ID NO: 21) Reverse primer: 5'-AAAGTCAGATTTGTTGCTCCA-3' (SEQ ID NO: 22)

### 7) Construction of TCRβ-p2A-TCRα vector (TCR donor vector)

TCRs are heterodimers consisting of alpha and beta chains, and to express a functional TCR, both alpha and beta chains need to be expressed. In general, in such a case, the genes for the α and β chains are introduced and expressed at different positions in the genome. In this system, the foreign genes are introduced into only the TCR Dβ2 region, so the two genes must be expressed from a single site. The two main methods generally employed are a method using the internal ribosome entry site (IRES) and a method using the self-cleaving 2A peptide. IRES can produce two polypeptides from one mRNA, but the expression levels of two polypeptides could be biased. On the other hand, in the case of the method using a 2A peptide, one polypeptide is generated from one mRNA, which is cleaved to form two molecules, so the ratio of the two peptides will be one to one. In this example, both α- and β-chain genes were expressed simultaneously from the TCRDβ2 region by using a 2A peptide consisting of about 22 amino acids. Among several types of 2A peptides (p2A, T2A, E2A, F2A, etc.), the p2A peptide was used. The α and β chains linked by the p2A peptide are described as TCRα-p2A-TCRβ or TCRβ-p2A-TCRα.

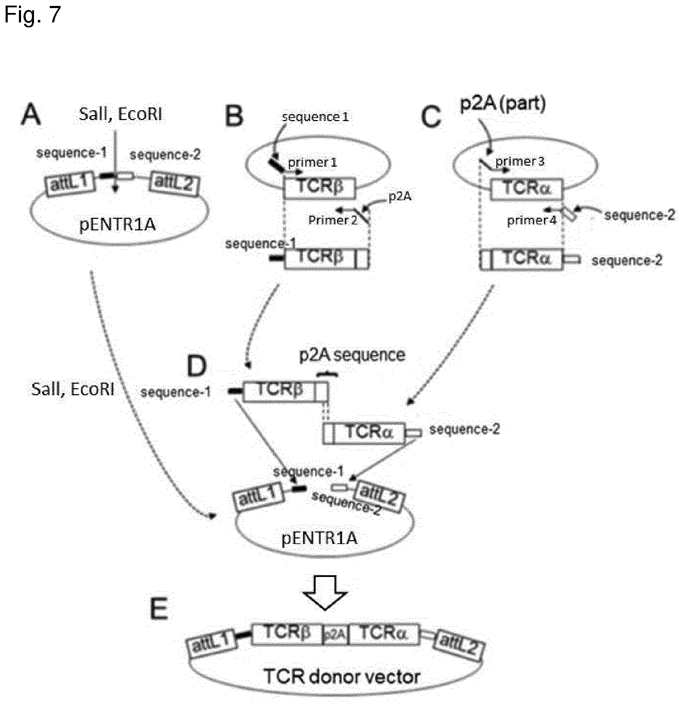

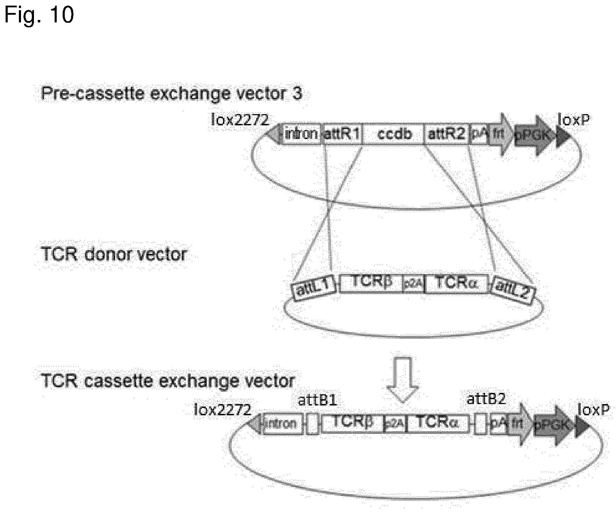

TCRβ-p2A-TCRα was constructed as follows. The pENTR vector is the entry vector (Thermo Fisher Scientific's Gateway system), which contains the attL sequence, the recombination sequence of the lambda phage, and the recombination between attL and attR is mediated by LR clonase recombinase (Fig. 10). The Gateway vector such as pENTR1A or pENTR3C (Thermo Fisher Scientific) was cut with restriction enzymes (SalI and EcoRI) as shown in Fig. 7A. On the other hand, plasmids with TCRβ (Fig. 7B) or TCRα (Fig. 7C) were used as templates to produce the respective DNA fragments by PCR. Primer 1 (Fig. 7B) had a portion of the TCRβ sequence and a sequence identical to 15bp of sequence-1 on the 5' side of the restriction enzyme cleavage site of the vector (Fig. 7A), and Primer 2 had a part of the TCRβ sequence and 54bp of the approximately 66bp DNA sequence of the p2A peptide. Primer 3 (Fig. 7C) had a part of the TCRα sequence and 28bp of the p2A sequence, and primer 4 had a part of the TCRα sequence and 15bp of the same sequence (sequence-2) on the 3' side of the restriction enzyme cleavage site of the vector. The pENTR1A vector cleaved with the restriction enzymes SalI and EcoRI, and the PCR products of TCRβ and TCRα, respectively, were linked by Gibson assembly method (Fig. 7D) through the parts overlapping each other (Fig. 7E).

When pENTR1A or pENTR3C is used as a vector and is cleaved with SalI and EcoRI, primer 2 and primer 4 can be used to clone all human TCRβ and TCRα into the vector.

The sequences of Primers were as follows, and PCR was performed in KOD-Plus-Neo at 1) 94°C, 2min→2) 98°C, 10sec→3) 50 to 60°C, 30sec→4) 68°C, 2min, and 2) to 4) for 30 to 35 cycles.
Primer 1: 5'-AAGGAACCAATTCAGTCGACCACCATGCTGCTGCTTCTGCTGCTT-3'(SEQ ID NO: 23)
Primer 2: 5'- 
CTCCTCCACGTCTCCAGCCTGCTTCAGCAGGCTGAAGTTAGTAGCTCCGCTTCCGCCTCTGG
AATCCTTTCTCTT-3′(SEQ ID NO: 24)
Primer 3: 5'-TGGAGACGTGGAGGAGAACCCTGGACCTATGATGATATCCTTGAGAGTT-3'(SEQ ID NO: 25)
Primer 4: 5'-TCGAGTGCGGCCGCGAATTCTCAGCTGGACCACAGCCGCAG-3'(SEQ ID NO: 26)

### 8) Construction of cassette exchange vector I: Preparation of vector framework

A plasmid (pBRBlII-AscI_FRTPGKpacΔtkpA_AscI) in which the Frt and PGK promoters were located near each other was used as the template to amplify the frt-PGK promoter DNA fragment by PCR (Fig. 8A). Primer 1 has a portion of the Frt sequence plus target sequences for restriction enzymes NheI and XhoI, lox2272 sequence, and a sequence identical to sequence-1 of the pBluescriptSK(-) vector (Fig. 8B), and primer 2 has a portion of the PGK promoter plus loxP sequence, and a sequence identical to sequence-2 of the pBluescriptSK(-) vector (Fig. 8A,B). Sequence-1 and sequence-2 of the pBluescriptSK(-) vector (Fig. 8B) are outside the restriction enzyme (KpnI and SacI) target sequences. The PCR products obtained using Primer 1 and Primer 2 were linked to the vector cleaved with restriction enzymes KpnI and SacI by Gibson assembly method to produce the Frt-PGK vector (Fig. 8C).

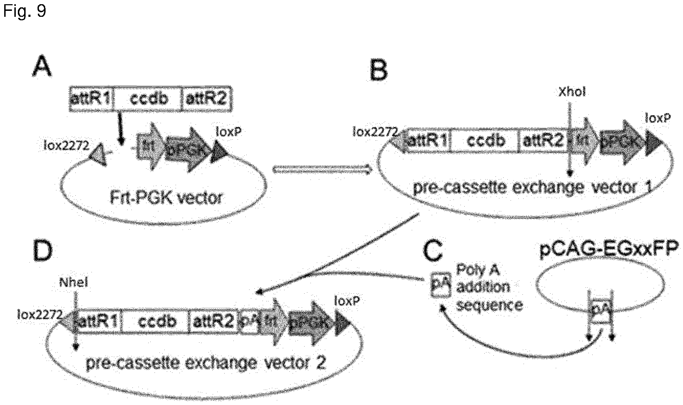

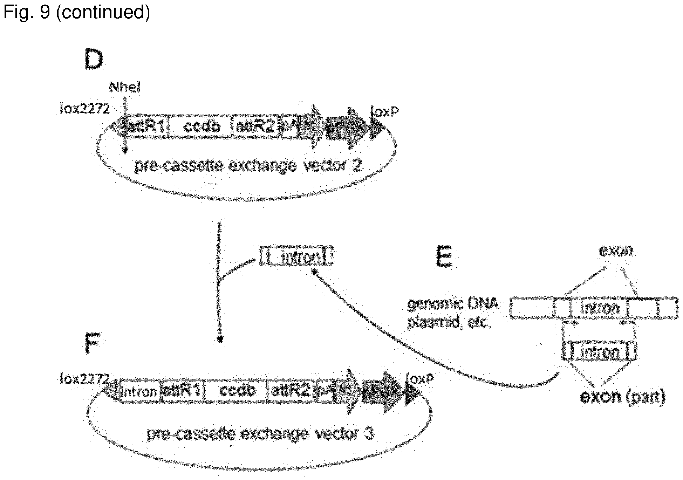

The Frt-PGK vector (Fig. 8C) was cleaved at the restriction enzyme recognition sites (NheI and XhoI) designed in primer 1, and DNA 1 with the cleavage end complementary to that of the restriction enzyme was linked to it using DNA ligase (Fig. 8E). Again, the vector was cleaved with one of the restriction enzyme recognition sites (NheI or XhoI) designed in primer 1, and was linked with DNA 2 in the same way as DNA 1. By repeating this process, multiple desired DNA fragments can be introduced between lox2272 and frt. The introduction can be done by DNA linkage reaction or Gibson assembly method. Using this method, the pre-cassette exchange vector was prepared (Fig. 9).

### 9) Construction of cassette exchange vector II: Construction of pre-cassette exchange vector

The gene cassette (RfA cassette) consisting of the attR1 sequence, chloramphenicol resistance gene, ccdb gene, and attR2 sequence was cut out from the CSIV-TRE-RfA-CMV-KT vector by restriction enzymes NheI and XhoI. The RfA cassette was ligated to Ftr-PGK vector (Fig. 9A) cleaved by restriction enzymes NheI and XhoI (Fig. 8D to 8G) by means of DNA ligase to construct a pre-cassette exchange vector 1 (Fig. 9B). A vector (pCAG-EGxxFP) was cleaved with XhoI to isolate the poly A-addition sequence of the rabbit β-globin gene (Fig. 9C) and introduced the gene into the XhoI-cleaved pre-cassette exchange vector 1 to construct the pre-cassette exchange vector 2 (Fig. 9D). Furthermore, the pre-cassette exchange vector 2 was cleaved with NheI, and the first intron of the human polypeptide chain elongation factor α (EF1α) or the intron of the chicken β-actin (CAG) gene promoter (Fig. 9E) amplified by PCR was linked by Gibson assembly method (Fig. 9F). The pre-cassette exchange vector with the intron immediately following lox2272 was designated as pre-cassette exchange vector 3.

The intron of the EF1α gene and the intron of the CAG promoter were isolated by PCR using human genomic DNA and pCAG-Cre-IP vector as templates and the following primers, respectively.
Intron in the EF1α gene:
F: 5'-TATACGAAGTTATCGCTAGCGGTTTGCCGCCAGAACACAG-3' (SEQ ID NO: 27)
R: 5'-TACAAACTTGTGATGCTAGCGTAGTTTTCACGACACCTGA-3' (SEQ ID NO: 28)
Intron in the CAG promoter:
F: 5'-TATACGAAGTTATCGCTAGCGCCCCGGCTCTGACTGACCG-3' (SEQ ID NO: 29)
R: 5'-TACAAACTTGTGATGCTAGCGACAGCACAATAACCAGCAC-3' (SEQ ID NO: 30)
PCR was performed in KOD-Plus-Neo at 1) 95°C for 3min, 2) 98°C for 10sec, 3) 50 to 60°C for 30sec, and 4) 68°C for 1 to 3min, followed by 30 to 35 cycles of 2) to 4).

### 10) Construction of cassette exchange vector III: Creation of TCR cassette exchange vector

Pre-cassette exchange vector 3 (Fig. 9F, upper part of Fig. 10) and TCR donor vector (Fig. 7E, middle part of Fig. 10) were mixed and reacted according to the manual of GatewayRL® Clonase™II enzyme mix (Thermo Fisher Scientific, 11791-020). In this reaction, recombination occurs between attR and attL, and the part flanked by attR1 and attR2 was replaced by the part flanked by attL1 and attL2 (Fig. 10). As a result, TCR cassette exchange vector (lower part of Fig. 10) was obtained. In this example, the pre-cassette exchange vector 3, which had the intron derived from the CAG promoter was used.

### 11) TCR cassette exchange in TCRα-KO drug resistance gene KI-Jurkat cells (Generation of TCR cassette exchange Jurkat cells)

### Materials

**[Table 4]**

| | | |
|---|---|---|
| Cell | TCRα-KO drug resistance gene KI-Jurkat cells | 2x10⁶ cells |
| Vectors | TCR cassette exchange vector | 1 µg |
| | Cre recombinase expression vector (pCAG-nls-Cre) | 4 µg |
| Reagent | Amaxa®CellLineNucleofector®KitV | 100 µL |

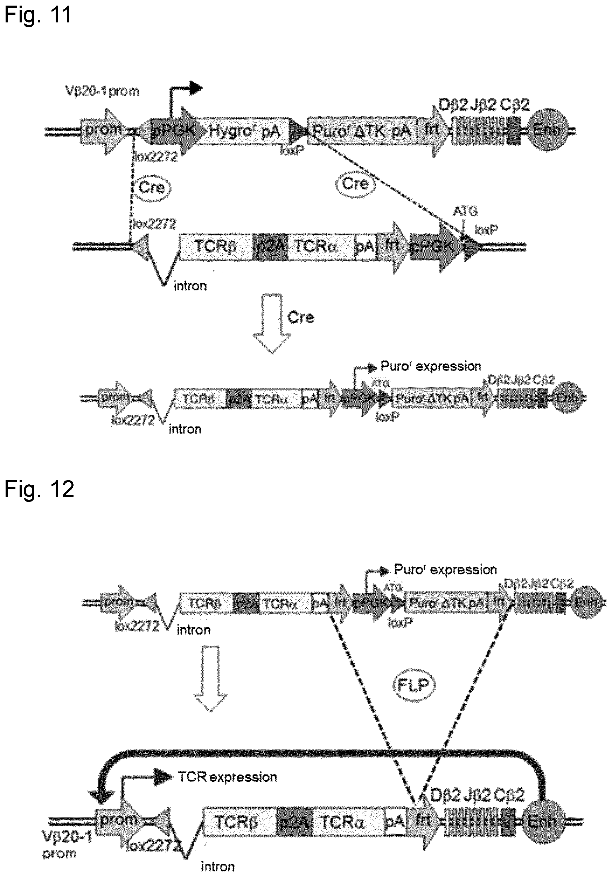

Fig. 11 schematically shows the Dβ2 region of the human TCRβ locus after homologous recombination (empty cassette deck: the region flanked by lox2272 and loxP) (top panel), the TCR cassette exchange vector (middle panel), and the TCRβ locus after the cassette exchange (bottom panel). To induce cassette exchange, TCR cassette exchange vector and Cre expression vector (pCAG-nls-Cre) were introduced into TCRα-KO drug resistance gene KI-Jurkat cells by electroporation. In this example, the TCR (KM#3-3) specific to WT1 antigen was introduced. With the introduction of Cre and the cassette exchange vector, the portion between the lox2272 and loxP, the hygromycin resistance gene and the TCR gene-frt-PGK promoter, were replaced by the action of Cre recombinase (cassette exchange). Before cassette exchange, the puromycin resistance gene was not expressed because it lacked the promoter and start codon. In contrast, after the cassette exchange, the PGK promoter was located immediately before the puromycin resistance gene, and the start codon placed immediately before loxP was linked to the puromycin resistance gene in the same translation reading frame. Thereby, expression of the puromycin resistance gene was initiated only in the cells in which the cassette exchange was successfully occurred. Cells were selected using 0.25 µg/mL puromycin/culture medium, and after about a week, puromycin-resistant cells (TCR cassette-exchanged Jurkat cells) were obtained.

### 12) Introduction of FLP vector into TCR cassette-exchanged Jurkat cells (creation of exogenous TCR-expressing Jurkat cells)

### Materials

**[Table 5]**

| Cell | TCR cassette exchanged Jurkat cell | 2x10⁶ cells |
|---|---|---|
| Vector | FLP recombinase expression vector (pCAGGS-FLPe) | 5 µg |
| Reagent | Amaxa®CellLineNucleofector®KitV | 100 µL |

The FLP recombinase expression vector (pCAGGS-FLPe) was introduced into TCR cassette-exchanged Jurkat cells by electroporation. FLP recognizes frt sequences and delete the region flanked by them. Fig. 12 shows the Dβ2 region of the TCRβ gene locus after the cassette exchange (upper panel) and the TCRβ gene locus with deletion of the portion flanked by frt sequences (lower panel).

A few days after the FLP introduction, the cells were stained with fluorescently labeled antibodies against TCR or CD3 (PE/Cy7anti-humanTCRα/β (306719, BioLegend) and APC-MouseAnti-Human CD3 (557597, BDPharmingen)) and analyzed by FACS to evaluate the expression level of the exogenous TCR. In T cells in vivo, the promoter of the TCRVβ gene is regulated by enhancer of the TCRCβ region. Therefore, it can be expected that the use of the promoter of the TCRVβ gene in this example will result in the regulation similar to that under physiological conditions. After the FLP introduction, the expression of the exogenous TCR was actually observed (Fig. 13). It is thought that the deletion between the Frt sequences occurred and the enhancer (Enh) could act efficiently on the promoter (prom), resulting in the expression of the TCR.

### 13) Removal of non-FLP recombinant cells by ganciclovir

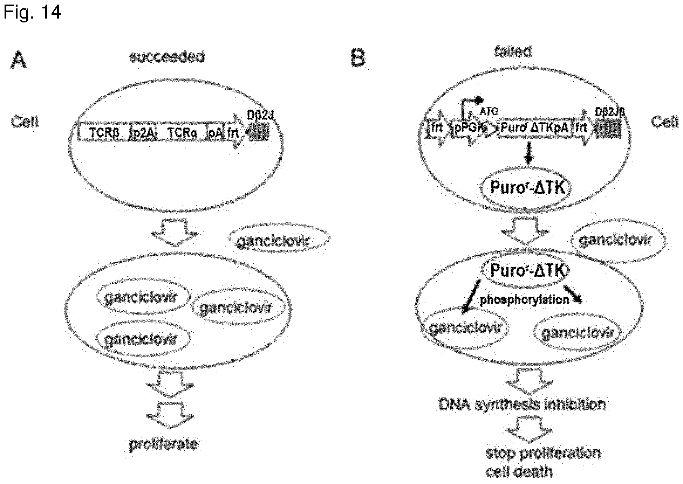

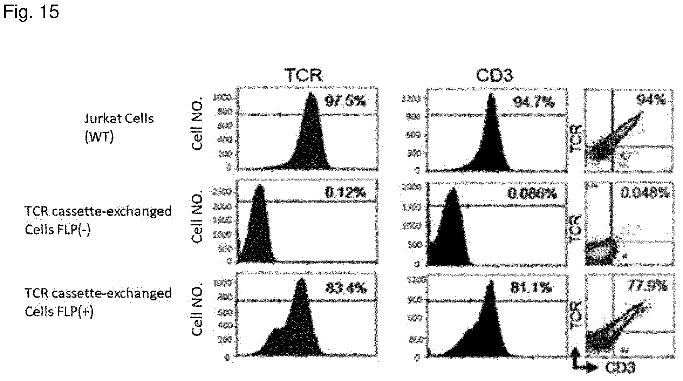

In the result shown in Fig. 13, there were two populations, one in which the TCR was expressed and one in which it was not. It is possible that the cells in which the region flanked by frt was deleted by the action of FLP while cells in which no deletion occurred were mixed. Therefore, the cells that failed to be deleted the region (cells that failed to be recombined by FLP) were removed by using ganciclovir. Normally, ganciclovir does not act on human cells such as Jurkat cells (Fig. 14A). In contrast, in the cells with PurorΔTK, ganciclovir becomes an analog of nucleic acid when phosphorylated by ΔTK, resulting in inhibition of DNA replication, to cause arrest of cell proliferation, or cell death (Fig. 14B). The expression of TCR and CD3 on the cell membrane was analyzed by FACS after 5 days of incubation with 12 µM ganciclovir/culture medium. As a result, almost no cells that did not express TCR were found (Fig. 15). The cell populations that did not express TCR are considered to have not undergone recombination by FLP.

### [EXAMPLE 2]

### Knocking-in of a drug resistance gene cassette into the Dβ2 region of the TCRβ locus in human iPS cells

### 1) Reagents, antibodies, etc:

Stem Fit®AK02N (TAKARA, AJ100), Y-27632 (Wako, 257-00511), Vitronectin (Thermo Fisher, A14700), Hygromycin (Invivogen, ant-hg-1), lipofectamine® (Thermo Fisher, 11668027), Opti-MEM® (Thermo Fisher, 31985070) and KOD-FX (TOYOBO, KFX-101) were used.

As human iPS cells, 253G1 (RIKEN, derived from human skin cells) was used.

### 2) Knocking-in of a drug resistance gene cassette into the Dβ2 region of the TCRβ locus in human iPS cells by homologous recombination

### Isolation of drug resistance gene knock-in (KI)-iPS cells

In all experiments, Stem Fit® AK02N (TAKARA, AJ100), a medium for human iPS cells, was used for iPS cell culture. Y-27632 (Wako, 257-00511) was added to Stem Fit® at a final concentration of 10 µM. Cells were seeded in 6-well plates coated with vitronectin (Thermo Fisher, A14700). When selecting with hygromycin, 50 µg/mL of Hygromycin (Invivogen, ant-hg-1) was added to the culture.

Drug resistance gene cassette knock-in targeting vector was designed in the same way as in Example 1 and introduced into iPS cells in the same manner as in Example 1. Lipofection method was used to introduce the vector into the cells. Lipofection was performed according to the manual of lipofectamine® (Thermo Fisher, 11668027). The vector and lipofectamine® were suspended in Opti-MEM® (Thermo Fisher, 31985070) medium and mixed with the cells to introduce the vector into the cells.

The Vβ20-1 promoter and drug resistance gene cassette were introduced into the approximately 50 bp upstream of the Dβ2 gene in the TCRβ locus (Fig. 6,upper panel) of human iPS cells using the drug resistance gene KI targeting vector (Fig. 6, middle panel). A schematic diagram of a successful introduction is shown in Fig. 6, lower panel.

To increase the efficiency of the knock-in by homologous recombination, a single double-strand break was introduced at a site approximately 50bp upstream of the Dβ2 gene by the CRISPR/Cas9 system. KI targeting vector was introduced into human iPS cells along with CRISPR/Cas9 vector for double-strand break introduction (see material below).

In the cells that have been incorporated with the drug resistance gene cassette into their genomic DNA by homologous recombination, the hygromycin resistance gene, the first drug resistance gene, is expressed by the action of the PGK promoter (the puromycin resistance gene, the second drug resistance gene, is not expressed here) (Fig. 6, lower panel). For this purpose, clones that incorporated the drug resistance gene cassette into their genome were selected using 50 µg/mL hygromycin/culture medium (positive selection).

On the other hand, the cells into which the outer portion of the 5' arm and 3' arm of the drug resistance gene KI targeting vector was incorporated by random integration cannot survive because diphtheria toxin (DTA) (Fig. 3) is produced intracellularly (negative selection). Then, from the cells (clones) so selected, clones that were incorporated with the part from the vβ20-1 promoter to frt of the drug resistance gene cassette into the TCRDβ2 locus were identified by PCR. Thus, the clone with the vβ20-1 promoter and drug resistance gene cassette in the Dβ2 region was selected (drug resistance gene KI-iPS cells).

### Materials

**[Table 6]**

| Cell | human iPS cells | 1x10⁵cells |
|---|---|---|
| Vectors | drug resistance gene KI targeting vector | 1.4µg |
| | CRISPR/Cas9n vector | 0.7µg |
| Reagents | Lipofectamine® | 2µL |
| | Opti-MEM® | 50µL |

The CRISPR/Cas9 vector was prepared by using the following oligonucleotides A and B:
A: 5'-CACCCTGCCGCTGCCCAGTGGTTG-3' (SEQ ID NO: 31)
B: 5'-AAACCAACCACTGGGCAGCGGCAG-3' (SEQ ID NO: 32)
Oligonucleotides A and B were annealed and introduced into plasmid pX330, which was cleaved with restriction enzyme BbsI.

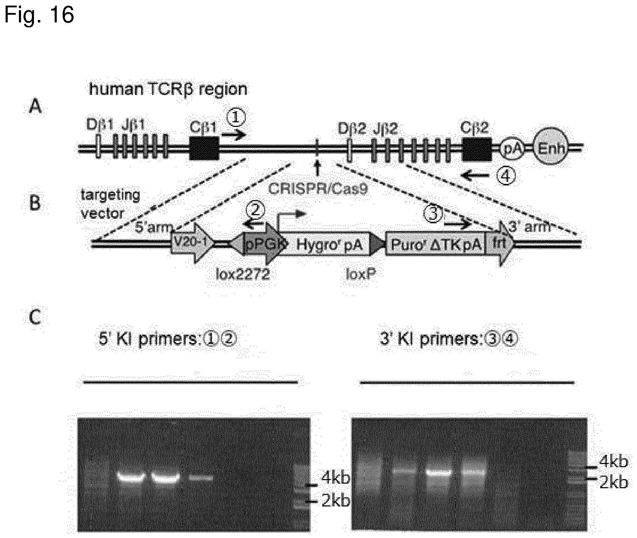

PCR was performed on the hygromycin-resistant clones using genomic DNA as the template to check for successful knock-in. PCR was performed using KOD-FX (TOYOBO, KFX-101) at 1) 94°C for 2 min, → 2) 98°C for 10 sec, → 3) 60°C for 30 sec, → 4) 68°C for 5 min, with 35 cycles of 2) to 4). The primers used in the PCR are shown below.
Primer Forward (1)5'-ACGGCTGAAATCTCCCTAACCC-3'(SEQ ID NO: 33)
Primer Reverse (2)5'-TACTTCCATTTGTCACGTCCTG-3'(SEQ ID NO: 34)
Primer Forward (3)5'-CCTGCTGCAACTTACCTCC-3'(SEQ ID NO: 35)
Primer Reverse (4)5'-GGGGACCGAGGGGCTGGAAG-3'(SEQ ID NO: 36)
The sequence sites of each primer are shown in Fig. 16A and (B). The results of electrophoresis of PCR products are shown in Fig. 16C. In Fig. 16C, 1, 2, and 3 indicate clone numbers. Clone2 and 3 were confirmed to be the clones that were successfully knocked in correctly. Genomic DNA from KI-Jurkat cells and pre-knock-in iPS cells were used as Positive Control (PC) WTiPS respectively.

### [Example 3]

This is an example of the method shown in Fig. 2-1, i.e., introducing a cassette for introducing a gene containing an exogenous TCR or CAR gene so as to reduce the distance between the C region enhancer of a TCR and a V region promoter in of a TCR in the material cell.

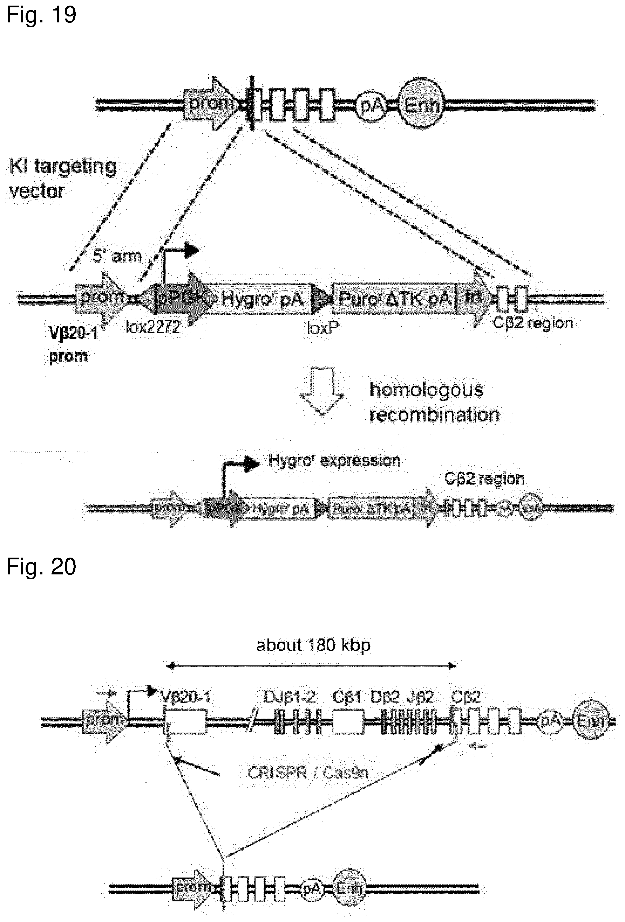

In Example 3, a cell line in which a 180-kbp region was deleted from the vβ20-1 gene in the V region to the Cβ2 gene in the C region of the TCRβ locus where no major genetic rearrangement had occurred was established. A schematic diagram of the method for establishing the clone is shown in Fig. 20. As material cells, a variant clone of the Jurkat cell line, the J.RT3-T3.5 Jurkat cell line (hereafter referred to as Jurkat β mutant), in which the rearranged TCRβ gene has no longer been expressed due to the introduced mutation (Ohashi et al., Science 316. 606-609. 1985) was used.

### (1) Genomic DNA truncation at the vβ20-1 and Cβ2 regions in the TCRβ locus.

The genomic DNA of Jurkat β mutant cells was cut by introducing two single-strand breaks (nicks) in the region 30 bp to 80 bp downstream from the translation start point of the TCRVβ20-1 gene and in exon 1 of the TCRCβ2 gene, respectively (vertical lines in Fig. 20, upper panel). For genomic DNA cleavage, four CRISPR/Cas9n vectors (see materials below) and EGFP expression vector were both introduced into Jurkat β mutant by electroporation.

### Materials

**[Table 7]**

| Cells | Jurkat β mutant | 1 x 10⁶ cells |
|---|---|---|
| Vectors | 1. CRISPR/Cas9n vector 1 | 0.625 µg |
| | 2. CRISPR/Cas9n vector 2 | 0.625 µg |
| | 3. CRISPR/Cas9n vector 3 | 0.625 µg |
| | 4. CRISPR/Cas9n vector 4 | 0.625 µg |
| | 5. pmax-GFP | 0.25 µg |
| Reagent | Amaxa®CellLineNucleofector®KitV | 100µL |

Vectors for CRISPR/Cas9n were as follows:
vβ20-1 side
   A: 5'-CACCGGTAGAAGGAGGCTTATACC-3'(SEQ ID NO: 37)
   B: 5'-AAACGGTATAAGCCTCCTTCTACC-3'(SEQ ID NO: 38)
   C: 5'-CACCGGGTGGGCATGTGCGTGTGT-3'(SEQ ID NO: 39)
   D: 5'-AAACACACACGCACATGCCCACCC-3'(SEQ ID NO: 40)
Cβ2 side
   E: 5'-CACCGCAAACACAGCGACCTCGGGT-3'(SEQ ID NO: 41)
   F: 5'-AAACACCCGAGGTCGCTGTGTTTGC-3'(SEQ ID NO: 42)
   G: 5'-CACCAGAGATCTCCCACACCCAAA-3'(SEQ ID NO: 43)
   H: 5'-AAACTTTGGGTGTGGGAGATCTCT-3' (SEQ ID NO: 44)

Oligonucleotides A and B (vector 1), C and D (vector 2), E and F (vector 3) and G and H (vector 4) were annealed and introduced into plasmid pX460, which was cleaved with restriction enzyme BbsI.

### (2) Isolation of the cells lacking the 180 kbp region from the vβ20-1 gene to the Cβ2 gene.

In the cells with more CRISPR/Cas9n vectors incorporated, genome cleavage is expected to occur with higher efficiency. Therefore, a group of cells that incorporated a particularly large number of vectors was selected by sorting and cloned. Two days after the gene transfer, a group of cells with particularly high expression levels of EGFP, an indicator of gene transfer efficiency, was isolated using a cell sorter and seeded directly into 96-well plates with one cell per well and cultured (cloning) (Fig. 21A). To maintain the survival of the Jurkat β mutant cells, 96-well plates were pre-seeded with 1 x 10⁵ B6 mouse thymocytes. Four to five weeks after the start of the culture, genomic DNA was extracted from each clone and analyzed by PCR to see if the vβ20-1 and Cβ2 regions were linked. The primers used for the PCR are shown below. The forward primer is approximately 140 bp upstream of the vβ20-1 gene translation start site, and the reverse primer corresponds to the sequence immediately after exon 1 of the Cβ2 gene (Fig. 21B). If the linkage occurred in the vicinity of the site where cleavage was expected to occur upon introduction of the CRISPR/Cas9n vectors, a DNA fragment of approximately 500 bp could be amplified by PCR. Electrophoretic analysis of the PCR reaction products suggested linkage of the vβ20-1 and Cβ2 regions in clones #10, #11, #16, and #17 (Fig. 21C). For clone #10, analysis of the sequence of the DNA fragment obtained by PCR amplification confirmed that the vβ20-1 and Cβ2 regions were linked via 6 bp of DNA (Fig. 21D). This resulted in the establishment of the Jurkat β mutant strain, in which the region spanning approximately 180 kbp was deleted and the vβ20-1 and Cβ2 regions were linked. In other clones, the linkage of the vβ20-1 region to the Cβ2 region was also confirmed by DNA sequence analysis.

### PCR Primers

Forward primer: 5'-GTCATGGGCAAAGATTACCAC-3'(SEQ ID NO: 45)
Reverse Primer: 5'-GGTAGCTGGTCTCACCTAATC-3'(SEQ ID NO: 46)

By introducing a single-strand break between the C region enhancer and the V region promoter in a TCR in the cells obtained in this example that were modified to reduce the distance between the C region enhancer and the V region promoter, and then introducing an exogenous TCR or CAR gene into the cells, it is possible to produce cells in which the antigen receptor gene has been introduced.

### [Example 4]

### 1) Knocking-in of a drug resistance gene cassette deck into the Dβ2 region of the TCRβ gene locus in human iPS cells

The cassette deck sequence was knocked into human iPS cells (derived from a non-T cell, not genetically rearranged) by lipofection. The obtained cells were selected based on the drug resistance and cloned by colony picking to obtain cassette deck knocked-in iPS cells (cKI-iPSC).

### Materials

**[Table 8]**

| Cells | iPS cell line I14s03 (obtained from Center for iPS Cell Research and Application, Kyoto University) | 3.6×10⁵cells |
|---|---|---|
| Vectors | 1. pX330-hTCRDβ2-gRNA #1 (Crispr/CAS9 and guide RNA expression vector) | 0.7µg |
| | 2. HygroPuroDβ2-5' & V20-1 for Targeting | 1.4µg |
| | drug resistance gene cassette deck | |
| | KI targeting vector (Fig. 22) | |
| Reagents | FuGENE HD (Promega) | 8µl |
| | Opti-MEM® (Thermo Fisher) | 100µl |
| | iMatrix-511 (Nippi) | 9.6µl |
| | Hygromycin B solution (Nakarai) | |
| medium | AK03N (Ajinomoto CO.,INC) | |

AK03N was used for culturing iPS cells in all experiments. When culturing single cell suspension of the iPS cells, Y-27632 (Wako) was added to give a final concentration of 10 µM. A 6-well plate coated with iMatrix (nippi) was used for culturing the cells.

### 1-1) Knocking-in of the targeting vector into the iPS cells

The iPS cells were seeded on the plate the day before the lipofection was performed. A mixture of the Crispr/CAS9 and guide RNA expression vector and the knock-in targeting vector in the above ratio were transferred to the iPS cells by lipofection. After suspending the vectors and the reagents in in Opti-MEM®, they were added onto the iPS cells, and the medium was exchanged 4-6 hours later.

### 1-2) Hygromycin selection

From 2 days later, drug selection was performed using hygromycin at the final concentration as shown below. In the cells in which the drug resistance gene cassette was incorporated into their genomic DNA by homologous recombination, the first drug resistance gene, hygromycin resistance gene, was expressed by the action of the PGK promoter while the second drug resistance gene, puromycin resistance gene, was not expressed at this stage. Therefore, a clone in which the drug resistance gene cassette was incorporated into the genome was first selected using the medium containing hygromycin (positive selection).

Day 0: lipofection
Day 1: medium exchange
Day 2: hygromycin 25 µg/ml
Day 3: hygromycin 40 µg/ml
Day 4 - 6: hygromycin 50 µg/ml
Day 7: pick-up iPS colonies

### 1-3) Establishment of the cassette knocked-in iPS cells (cKI)

After picking up the iPS cell colonies that remained after the drug selection, 5 clones were established. DNA was collected from each clone and PCR was performed to confirm the clone of interest. Cells in which the outer portion of the 5' arm and 3' arm of the drug resistance gene KI targeting vector was incorporated into the genomic DNA by random integration could not survive because diphtheria toxin (DTA) was produced in the cells and were removed (negative selection). Next, from the cells (clones) selected in this way, a clone in which the part from the vβ20-1 promoter to frt of the drug resistance gene cassette was incorporated into the TCRDβ2 gene locus was identified by PCR. Based on the above, a clone (cKI-iPSC) in which the vβ20-1 promoter and drug resistance gene cassette were introduced into the Dβ2 region was selected.

### 2) "Cassette tape exchange" on the iPS cells

On the genome of cKI-iPSC established in step 1), the exogenous TCR was introduced in the manner of cassette exchange using the first recombinase.

### Materials

**[Table 9]**

| Cells | cKI-iPS cell derived from strain I14s03 | 1×10⁶ cells |
|---|---|---|
| Vectors | 1. pCAGGS-NLS-Cre (Cre recombinase expression vector) | 7.9 µg |
| | 2. CAGint-WT1W3-3-EF1a (Cassette exchange vector) | 2.4 µg |
| Regents | Opti-MEM (Thermo Fisher) | 100 µl |
| | iMatrix-511 (nippi) | 9.6 µl |
| | Puromycin (Gibco) | |
| Device | NEPA21 (Nepa Gene Co., Ltd.) | |
| Medium | AK03N (Ajinomoto CO.,INC) | |

AK03N was used for culturing iPS cells in all experiments. When culturing single cell suspension of the iPS cells, Y-27632 (Wako) was added to a final concentration of 10 µM. A 6-well plate coated with iMatrix (nippi) was used for culturing the cells.

2-1) The cKI-iPS cells were transfected with the mixture of Cre expression vector and cassette exchange vector in the above proportion by electroporation. The cKI-iPS cells were collected and converted into a single cell suspension, and the above number of the cells were suspended in Opti-MEM® with the vectors to give a 100 µl suspension, and then, electroporation was performed. Immediately after the electroporation, the cells were seeded on the 6-well plates and cultured.

### 2-2) Puromycin drug selection

There was a second promoter downstream of the cassette introduced in step 1), and when the cassette is successfully exchanged, the puromycin resistance gene downstream of the promoter will work in the iPS cells. By this mechanism, iPS cells in which TCR/CAR cassette exchange was successfully occurred are resistant to puromycin. Therefore, the drug was used to select the cells in the final concentration shown below. The cells were harvested 2 days after the electroporation and reseeded to give cultures with the same number of the cells, and then, subjected to the puromycin selection. This is because efficiency of the selection by puromycin is significantly vary depending on the cell density in the case of iPS cells.

Day 0: Electroporation
Day 1: medium exchange
Day 2: reseeding the cells at 5×10⁴ cells/well
Day 3: puromycin 180 ng/ml
Day 4: puromycin 150 ng/ml
Day 5-9: medium exchange
Day10: pick up iPSC colonies

### 2-3) Establishment of cassette tape exchanged iPS cells (exTCR-KI-iPSC)

After picking up the iPS cell colonies that survived the drug selection, four clones were established. DNA was collected from each clone and PCR was performed to confirm the clone of interest. A 5'side confirming primer sandwiching the upstream of the 5' arm and the CAG intron of the cassette vector and a 3' side confirming primer sandwiching the downstream of 3' arm and the EF1α promoter in the vector were used. A strain in which both bands could be detected was established as the cassette exchanged TCR knock-in iPS cells (exTCR-KI-iPSC).

### 3) Deletion of the PurorΔTK

On the genome of exTCR-KI-iPSC established in step 2), the PurorΔTK site was deleted using the second recombinase, and the production of the T cell-producing iPS cells was finally completed.

### Materials

**[Table 10]**

| Cells | exTCR-KI-iPS cells, derived from strain I14s03 | 1×10⁶ cells |
|---|---|---|
| Vector | pCAGGS-FLP | 10 µg |
| Reagents | Opti-MEM (Thermo Fisher) | 100 µl |
| | iMatrix-511 (nippi) | 9.6 µl |
| | Ganciclovir (Wako) | |
| Device | NEPA21 (Nepa Gene Co., Ltd.) | |
| Medium | AK03N (Ajinomoto CO.,INC) | |

AK03N was used for culturing iPS cells in all experiments. When culturing single cell suspension of the iPS cells, Y-27632 (Wako) was added to a final concentration of 10 µM. A 6-well plate coated with iMatrix (nippi) was used for culturing the cells.

### 3-1) Transfection of FLP plasmid into the exTCR-KI-iPS cells and removal of the region sandwiched by frt

Single cell suspension of the cells was prepared and the above number of the cells were suspended in Opti-MEM® with the plasmid vector to give a 100 µl suspension, and then, electroporation was performed. After the electroporation, the cells were immediately seeded and cultured in 6-well plates.

### 3-2) Ganciclovir drug selection

Due to the action of thymidine kinase downstream of the cassette vector established in step 2), ganciclovir (GCV) became toxic in the cells, and therefore, the cells die when co-cultured with GCV. Using this mechanism, GCV agent at the final concentration shown below was used to select cells. Selection by the drug was started the day after the cells were harvested 2 days after electroporation and reseeded with the same number of cells. This is because efficiency of the selection by GCV is significantly vary depending on the cell density in the case of iPS cells.

Day 0: electroporation
Day 1: medium exchange
Day 2: reseeding the cells at 1×10⁵ cells /well
Day 3-13: GCV 5µg/ml
Day 14: pick up the iPSC colonies

### 3-3) Cassette tape exchanged iPS cells with deleted PurorΔTK (final product)

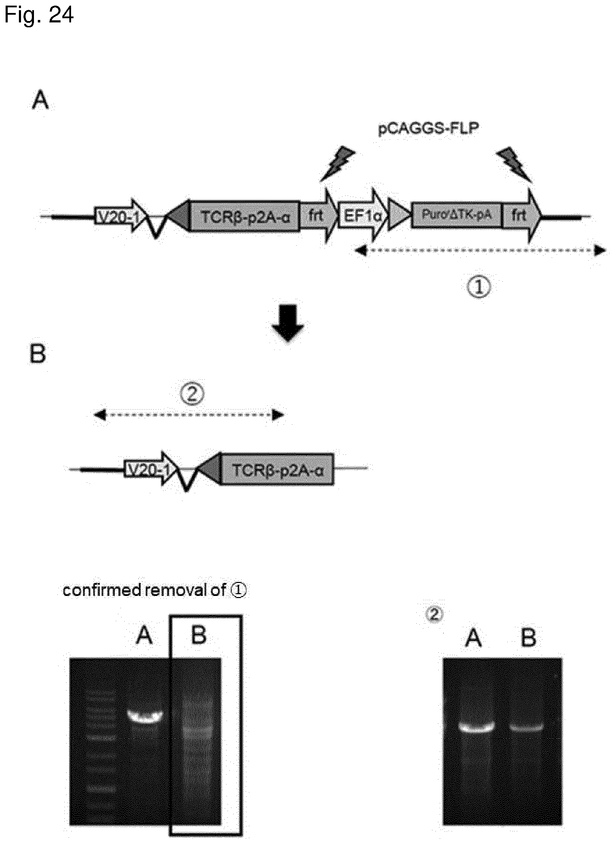

After picking up the iPS cell colonies that survived after drug selection, two clones were established. DNA was collected from each clone and PCR was performed to confirm the clone of interest. Fig. 24 shows the results of PCR using the primers sandwiching EF1α promoter site at the site to be removed and the downstream of the 3'arm. The TCR-KI-iPS cells that were successfully exchanged the cassette, the band of PurorΔTK could not be confirmed. (Fig. 24, bottom, B lane)

### [EXAMPLE 5]

### Cytotoxic activity of regenerated CTLs prepared by differentiating the NY-ESO1-specific TCR-KI-iPS cells (final product) into T cells

iPS cells in which NY-ESO1-specific TCR was knocked in were obtained by the method of Example 4. The obtained iPS cells were differentiated into T cells, and the cytotoxic activities of the obtained regenerated CTLs against cancer cell lines were evaluated.

The NY-ESO1-TCR-KI-iPS cell-derived regenerated CTLs were CTL cells derived from iPS cells by knocking in NY-EOS1-specific TCR into the cells by the method of Example 4. Induction of CTLs from NY-EOS1-KI-iPS cells was performed by the method described in WO 2017/179720 (US20190161727) (this document is incorporated herein by reference). iPS cells were induced into T cell precursors, which were CD4CD8 double positive cells, and the CD4CD8 double positive cells were isolated.

The isolated CD4CD8 double positive cells were further induced into CD8 single positive cells. As a result, CD8 single positive T cells in which the CD8 antigen was a heterozygous type of CD8 α and CD8 β chains were obtained (Fig. 25).

As reference cells, regenerated CTL cells differentiated from WT1-TiPS cells were used. The rearranged CTL cells were differentiated from T-iPS cells that were induced from T cells with a WT1-specific TCR. The induction of iPS cells from T cells having an antigen-specific TCR was based on the method described in WO 2016/010155 (US20170267972). Induction of CTLs maintaining the antigen specificity from iPS cells having antigen-specific TCR was carried out in the same manner as described above.

### (2) Evaluation of cytotoxic activity against multiple myeloma cell line U266 positive for A0201 and expressing NY-ESO1

Luciferase-introduced U266 (multiple myeloma cell line, NY-ESO1+, HLA-A02+), Bright-Glo (Promega) and Glomax (Promega) were used.

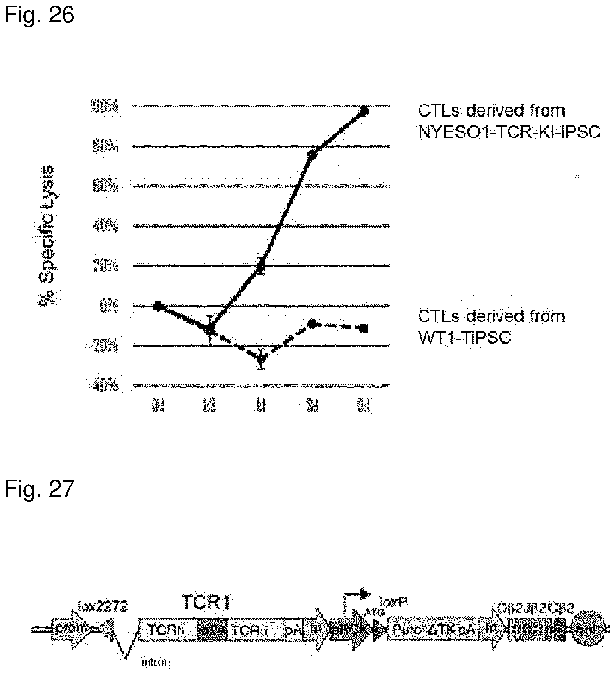

The cytotoxic activity of the CTLs regenerated from the NY-ESO1-specific TCR KI iPS cells against multiple myeloma cell line U266 was evaluated. As a comparison, the cytotoxic activity of CTLs regenerated from WT1-TiPS cells against the same cell line was simultaneously examined. The regenerated CTLs and U266 cells were mixed at the ratio of 0: 1, 1: 3, 1: 1, 3: 1, 9: 1, and then cultured in an environment of 37 °C and 5% CO₂ for 6 hours. After the culture, cytotoxic activities were evaluated based on the ratio of Annexin V positive cells. The results are shown in FIG 26.

The NY-ESO1-TCR-KI-iPS cell-derived regenerated CTLs showed cytotoxic activity against U266 cells in a cell number-dependent manner. The CTLs regenerated from the WT1-TiPS cells exerted almost no cytotoxic activity.

### [Example 6]

### Exchange of an exogenously introduced T cell receptor (TCR) gene in T cells with another TCR gene

By the same method as in Example 1 (Fig. 11), the drug resistance gene cassette knock-in vector was knocked into the TCRDβ2 region of the Jurkat cells, and then the TCR 1 gene was exchanged with the drug resistance gene cassette to obtain drug resistance gene introduced Jurkat cells. As the TCR 1 gene, a synthetic gene in which the α and β chains of TCR (KM # 3-3) that recognizes the WT1 antigen was linked by the p2A peptide gene was used. Fig. 27 shows a schematic diagram of the TCRβ gene locus DJ region of the Jurkat-TCR 1 cells of this example.

The TCR 1 gene in Jurkat-TCR 1 cells was then replaced with the TCR 2 gene by the method shown below. The TCR 2 gene was a synthetic gene in which the α and β chains of the TCR that recognizes the NYESO1 antigen were linked by the p2A peptide gene.

### Co-introduction of TCR 2 cassette exchange vector and Cre recombinase expression vector into Jurkat-TCR 1 cells and confirmation of the exchange of TCR 1 with TCR 2 in Jurkat cells by means of PCR

### Materials

**[Table 11]**

| Cells | Jurkat-TCR 1 cells | 2 x 10⁶ cells |
|---|---|---|
| Vectors | 1. TCR 2 cassette exchange vector | 1 µg |
| | 2. Cre recombinase expression vector (pCAG-nls-Cre) | 4 µg |
| Reagents | Amaxa® Cell Line Nucleofector® Kit V | 100 µL |

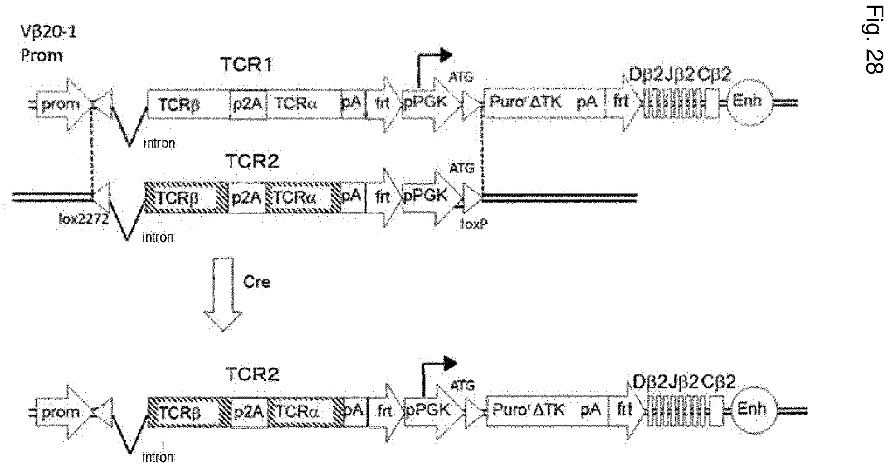

Fig. 28 provides schematic diagrams of the TCRβ locus DJ region (upper) in which TCR 1 is incorporated, the TCR 2 cassette exchange vector (middle), and the TCRβ locus after the cassette exchange (lower). In order to induce cassette exchange, TCR 2 cassette exchange vector and Cre recombinase expression vector (pCAG-nls-Cre) were introduced into Jurkat-TCR 1 cells by electroporation. With the introduction of the Cre recombinase and the cassette exchange vectors, the part sandwiched between lox2272 and loxP, that is, the TCR 1-frt-PGK promoter is replaced with the TCR 2 gene-frt-PGK promoter by the action of the Cre recombinase is expected (cassette exchange, Fig. 28).

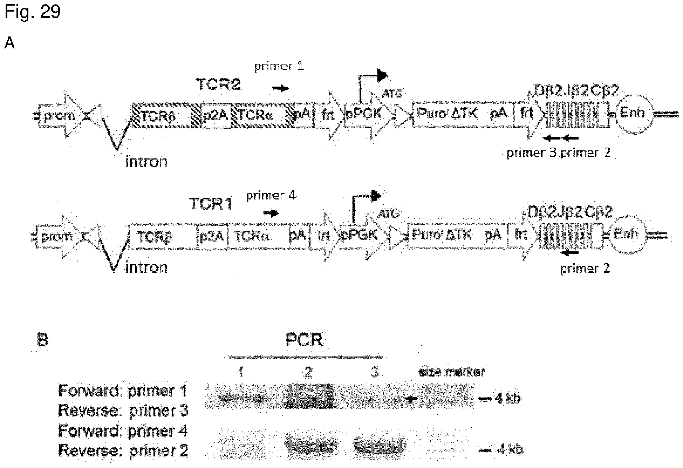

Genomic DNA was extracted from the cells 5 days after the transfection and analyzed by PCR to see if the exchange of TCR1 with TCR 2 occurred (Fig. 29). In addition, as a control experiment for the PCR reaction, the genomic DNA of Jurkat cells into which NYESO1-TCR (TCR 2) or KM # 3-3-TCR (TCR 1) was introduced by cassette exchange with the drug resistance gene were used for the PCR reaction. (Fig. 29B, PCR 1 and PCR 2). Primer 1 corresponds to TCR 2, primer 2 and primer 3 correspond to the DJ region, and primer 4 corresponds to TCR 1 (Fig. 29A). PCR was performed using KOD-FX (Toyobo, KFX-101) at 94°C, 2 min → 98°C, 10 sec, 61 °C, 30 sec, 68 °C, 3 mim in 30 to 35 cycles. The primers used in the PCR are shown below.

PCR was carried out using Primer 1 and Primer 2, and a 10-fold dilution of the reaction product was then subjected to PCR using Primer 1 and Primer 3. If the reaction to exchange TCR 1 with TCR 2 has occurred, it was expected that a DNA fragment of about 4 kb will be amplified. As a result of the electrophoresis, a band showing the exchange from TCR 1 to TCR 2 was observed (Fig. 3B, PCR3). On the other hand, amplification of the TCR 1 gene was expected when PCR was performed using primer 4 and primer 2. Amplification of the TCR 1 gene was observed in PCR2, and was also observed in PCR3 (Fig. 3B). From the results of PCR3, it is considered that the exchange from TCR 1 to TCR 2 occurred in some cells, but not in others.

From the above, it was shown that a TCR gene introduced exogenously in a T cell line Jurkat cells can be exchanged with another TCR gene.

### PCR Primers:

Primer 1, 5'-GGCAGCTACATCCCTACCTT-3' (SEQ ID NO: 47)
primer 2, 5'-CCACTTTGCTGTCTTGGCCTT-3'(SEQ ID NO: 48)
primer 3, 5'-AATCATCGTGCCCTCCCGCTA-3'(SEQ ID NO: 49)
primer 4, 5'-TCAGCCACCTACCTCTGTGT-3'(SEQ ID NO: 50)

## Claims

1. A method for producing a cell incorporating an antigen specific receptor gene, which comprises the step of introducing an exogenous TCR or CAR gene into a material cell so that the introduced gene is expressed under the T cell receptor expression control system of the material cell.

2. The method according to claim 1, wherein the T Cell receptor genes in the genome of the material cell have not been rearranged.

3. The method according to claim 1 or 2, wherein the material cell is a pluripotent stem cell.

4. The method according to any one of claims 1-3, wherein the exogenous TCR or CAR gene is introduced in the material cell by means of Recombinase-Mediate Cassette Exchange (RMCE) method or a genome editing method.

5. A material cell for introducing an exogenous antigen receptor gene, which comprises in a TCR locus in its genome, from upstream to downstream, a V region promoter of a TCR locus, a drug resistance gene or a reporter gene, or a known TCR or CAR gene, a C region enhancer of a TCR locus.

6. The material cell for introducing an exogenous antigen receptor gene according to claim 5, which comprises a target sequence (i) for a recombinase between the V region promoter in the TCR locus and the drug resistance gene or the reporter gene, or the known TCR or CAR gene, and a target sequence (ii) for the recombinase between the drug resistance gene or the reporter gene, or the exogenous TCR or CAR gene and the C region enhancer in the TCR locus.

7. The material cell for introducing an exogenous antigen receptor gene according to claim 5 or 6, wherein the cell has the known TCR or CAR gene.

8. A material cell for introducing an exogenous antigen receptor gene, which comprises in a TCR locus in its genome, from upstream to downstream, a V region promoter of a TCR locus, a drug resistance gene or reporter gene, or a known TCR or CAR gene, a target sequence (i) for a first recombinase, a promoter that can be expressed in the material cell, a first drug resistance gene a first drug resistance gene linked to be expressed under the promoter sequence that can be expressed in the material cell, a target sequence (ii) for the first recombinase that differs from the target sequence (i), a second drug resistance gene, a target sequence for a second recombinase, and a C region enhancer of a TCR locus.

9. The material cell according to any one of claims 5-8, wherein the TCR locus in the material cell is TCRα or TCRβ chain in the material cell.

10. The material cell according to claim 9, wherein the TCRα and TCRβ chains in the material cell other than the TCR chain in which the drug resistance gene or the reporter gene, or the exogenous known TCR or CAR gene is introduced are defected.

11. The material cell according to any one of claims 5-10, wherein the material cell is a pluripotent stem cell.

12. A method for producing the material cell according to any one of claims 8-11, which comprises the steps of:
(a) preparing a vector comprising a drug resistance gene cassette, which comprises in order from upstream to downstream, a V region promoter sequence of a TCR locus, a target sequence (i) for a first recombinase, a promoter sequence that can be expressed in the material cell, a first drug resistance gene linked to be expressed under the promoter sequence that can be expressed in the material cell, a target sequence (ii) for the first recombinase that differs from the target sequence (i) and a second drug resistance gene;
(b) knocking-in the vector prepared in step (a) in a TCR locus in the material cell genome; and
(c) selecting the cells to which the drug resistance gene cassette was successfully knocked in, comprising culturing the cells obtained in step (b) in the presence of the drug to which the first drug resistance gene is resistant.

13. The method according to claim 12, wherein in step (b), the vector prepared in step (a) is knocked in a site downstream of a V region promoter in the non-rearranged TCR locus so that the drug resistance gene cassette is replaced with the V region and DJ region downstream of the V region promoter.

14. The method according claim 12, wherein the vector prepared in step (a) further comprise a V region promoter in a TCR locus at the most upstream site, and in step (b), the vector prepared in step (a) is knocked into the DJ region of a TCR locus in the material cell genome.

15. The method according to claim 12, wherein the vector prepared in step (a) further comprises a C region enhancer in a TCR locus at the most downstream site, and in step (b), the vector prepared in step (a) is knocked into the DJ region of a TCR locus in the material cell genome.

16. A method for producing cells in which an exogenous antigen receptor gene is incorporated, which comprising the steps of:
(A) providing the material cells according to claim 8;
(B) preparing a TCR or CAR gene cassette exchange vector comprising, in order from upstream to downstream, a target sequence (i) for a first recombinase, an exogenous TCR or CAR gene, a target sequence for a second recombinase, a promoter that can be expressed in the material cell, and a target sequence (ii) for the first recombinase;
(C) introducing the TCR or CAR gene cassette exchange vector into the material cells, and simultaneously applying the first recombinase to exchange the drug resistance gene cassette with the sequence flanked by the target sequences (i) and (ii) for the first recombinase in the TCR or CAR gene cassette exchange vector;
(D) selecting cells that have successfully exchanged the cassette, comprising culturing the cells in the presence of a drug to which the second drug resistance gene is resistant; and
(E) causing the second recombinase to act on the cells selected in step (C) to remove the second drug resistance gene flanked by the target sequence for the second recombinase.

17. A method for producing cells in which an exogenous antigen receptor gene is incorporated, which comprising the steps of:
(1) preparing a vector comprising a drug resistance gene cassette comprising in order from upstream to downstream, a V region promoter in a TCR locus, a target sequence (i) for a first recombinase, a promoter that can be expressed in a material cell, a first drug resistance gene linked to be expressed under the promoter that can be expressed in the material cell, a target sequence (ii) for the first recombinase that differs from the target sequence (i), a second drug resistance gene, and a target sequence for the second recombinase,
(2) knocking-in the vector obtained in step (1) into a TCR locus on the material cell genome;
(3) culturing the cells obtained in step (2) in the presence of a drug to which the first drug resistance gene is resistant to select the cells to which the drug resistance gene cassette has successfully been knocked-in;
(4) preparing a TCR or CAR gene cassette exchange vector comprising, in order from upstream to downstream, the target sequence (i) for the first recombinase, an exogenous TCR or CAR gene, the target sequence for the second recombinase, a promoter that can be expressed in the material cell, and the target sequence (ii) for the first recombinase;
(5) introducing the TCR or CAR gene cassette exchange vector into the material cells selected in step (3) in which the drug resistance cassette has been knocked-in, and simultaneously applying the first recombinase to the material cells so that the sequence in the drug resistance gene cassette is replaced with the sequence flanked by the target sequences (i) and (ii) of the first recombinase in the TCR or CAR gene cassette exchange vector;
(6) applying the drug to which the second drug resistance gene is resistant to the cells to select the cells that has successfully exchanged the cassette; and
(7) applying the second recombinase on the cell selected in step (6) to remove the second drug resistance gene part flanked by the target sequence for the second recombinase.

18. The method according to claim 16 or 17, wherein the exogenous TCR or CAR gene is a rearranged TCR or CAR gene.

19. The method according to claim 16 or 17, wherein the exogenous TCR gene comprises both rearranged TCRα and TCRβ chains.

20. A method for producing cells for immune therapy, which comprising preparing the cells in which an exogenous TCR or CAR is incorporated by the method according to any one of claims 16-18, and differentiating the cells into T cells.

21. A method for producing cells for immune therapy, which comprising the steps of:
differentiating the material cells for introducing an exogenous antigen receptor gene according to any one of claims 5-7 into T progenitor cells or T cells, and introducing an exogenous TCR or CAR gene between the V region promoter and
the C region enhancer in the TCR on the genome of the T progenitor or T cells by means of Recombinase-mediated cassette exchange method or genome editing.

22. A method for producing cells for immune therapy, which comprising the steps of:
differentiating the material cells for introducing an exogenous antigen receptor gene according to claim 7 into T progenitor or T cells, and exchanging the known TCR or CAR gene in the T progenitor or T cells with an exogenous TCR or
CAR gene by means of Recombinase-mediated cassette exchange method (RMCE), and removing the cells which were unsuccessfully exchanged with the TCR or CAR gene by using an antibody or a tetramer specific for the known TCR or CAR.
